# EUROPEAN PATENT SPECIFICATION

(11) **EP 1 109 576 B1**
(45) Date of publication and mention of the grant of the patent: **21.10.2009**
(21) Application number: 99945115.6
(22) Date of filing: 18.08.1999
(51) Int. Cl.: A61K 39/385

(54) **IMMUNOGENIC BETA-PROPIONAMIDO-LINKED POLYSACCHARIDE PROTEIN CONJUGATE USEFUL AS A VACCINE PRODUCED USING AN N-ACRYLOYLATED POLYSACCHARIDE**
IMMUNOGENES BETA-PROPIONAMIDO-GEBUNDENES POLYSACCHARID-PROTEIN KONJUGAT GEEIGNET ALS IMPFSTOFF UND HERGESTELLT BEI VERWENDUNG VON N-ACRYLOYLIERTEM POLYSACCHARID
CONJUGUE DE PROTEINE-POLYSACCHARIDE IMMUNOGENE A LIAISON BETA-PROPIONAMIDO, UTILE COMME VACCIN ETABLI AU MOYEN D'UN POLYSACCHARIDE N-ACRYLOYLE

(30) Priority: 19.08.1998 US 97120 P; 18.08.1999 US 376911
(43) Date of publication of application: 27.06.2001
(73) Proprietor: Baxter Healthcare SA, 8306 Wallisellen (CH)
(72) Inventor: MICHON, Francis, Bethesda, MD 20814 (US); HUANG, Chun-Hsien, Bethesda, MD 20850 (US); UITZ, Catherine, Arlington, VA 22207 (US)
(74) Representative: Miles, John Stephen
(86) International application number: PCT/US1999/018982
(87) International publication number: WO 2000/010599

(56) References cited:
- WO-A-96/40239
- US-A- 4 356 170
- US-A- 4 727 136
- US-A- 5 576 002
- JENNINGS H J ET AL: "Induction of meningococcal group B polysaccharide-specific IgG antibodies in mice by using an N-propionylated B polysaccharide-tetanus toxoid conjugate vaccine." JOURNAL OF IMMUNOLOGY, (1986 SEP 1) 137 (5) 1708-13. , XP002052677
- JENNINGS H J ET AL: "Unique intermolecular bactericidal epitope involving the homosialopolysaccharide capsule on the cell surface of group B Neisseria meningitidis and Escherichia coli K1." JOURNAL OF IMMUNOLOGY, (1989 MAY 15) 142 (10) 3585-91. , XP000872571
- FUSCO P C ET AL: "Preclinical evaluation of a novel group B meningococcal conjugate vaccine that elicits bactericidal activity in both mice and nonhuman primates." JOURNAL OF INFECTIOUS DISEASES, (1997 FEB) 175 (2) 364-72. , XP000291371
- FUSCO, PETER C. ET AL: "Meningococcal vaccine development: a novel approach" EXPERT OPIN. INVEST. DRUGS (1998), 7(2), 245-252 , XP000872557
- GRANOFF D M ET AL: "Bactericidal monoclonal antibodies that define unique meningococcal B polysaccharide epitopes that do not cross-react with human polysialic acid." JOURNAL OF IMMUNOLOGY, (1998 MAY 15) 160 (10) 5028-36. , XP000872887
- JENNINGS H J ET AL: "Chemically modified Group B meninggococcal polysaccharides as human vaccines" PROCEEDINGS OF THE NATIONAL MEETING: SYMPOSIUM ON THE APPLICATIONS AND MODIFICATIONS OF INDUSTRIAL POLYSACCHARIDES,NL,AMSTERDAM, ELSEVIER, vol. MEETING 193, 1987, pages 149-156, XP002016901
- W.E. DICK ET AL.: "Glycoconjugates of Bacterial carbohydrate Antigens." CONJUGATE VACCINES, vol. 10, 1989, pages 48-114,

## Description

### FIELD OF THE INVENTION

The present invention relates to immunogenic β-propionamido-linked polysaccharide-protein conjugates and methods for producing the conjugates from bacteria, yeast, or cancer cells. The conjugates are useful as vaccines.

### BACKGROUND OF THE INVENTION

Bacterial infections caused by gram-positive bacteria such as Streptococcus, Staphylococcus, Enterococcus, Bacillus, Corynebacterium, Listeria, Erysipelothrix, and Clostridium and by gram-negative bacteria such as Haemophilus, Shigella, *Vibrio cholerae*, Neisseria and certain types of *Escherichia coli* cause serious morbidity throughout the world. This, coupled with the emerging resistance shown by bacteria to antibiotics, indicates the need for the development of bacterial vaccines. For example, streptococci are a large and varied genus of gram-positive bacteria which have been ordered into several groups based on the antigenicity and structure of their cell wall polysaccharide (26,27). Two of these groups have been associated with serious human infections. The group A streptococci cause a variety of infectious disorders including "strep throat", rheumatic fever, streptococcal impetigo, and sepsis. Group B streptococci are important perinatal pathogens in the United States as well as developing countries (37).

Gram-negative bacteria are also a significant cause of disease. Until the recent development and use of polysaccharide-protein vaccines directed against *Haemophilus influenzae* type b bacteria (Hib), Hib bacterial infections were responsible for many cases of mental retardation in infants. *N. menigitidis* and *E. coli* K1 infections are responsible for neonatal meningitis. Strains of gram-negative bacteria, E. *coli,* have been linked to serious illness including death from eating meat tainted with E. *coli* strains.

The polysaccharides have been used to elicit antibody responses to a variety of gram-negative and gram-positive bacteria, when conjugated to another immunogenic molecule such as a polypeptide or protein. Conjugation of the polysaccharide or oligosaccharide to the polypeptide converts the immune response to the polysaccharide or oligosaccharide which is typically T-cell independent to one which is T-cell dependent.

The prior art discloses both direct coupling and indirect coupling of polysaccharides to proteins to form conjugates (summarized in Ref (11) and U.S. Patent. No. 5,306,492). Conjugation methods have included diazo coupling, thioether bond, amidation, reductive amination and thiocarbamoyl for coupling a polysaccharide to a protein carrier.

Geyser et al., Med. Microbiol. Immunol, 165: 171-288 (1979) describes conjugates of certain *Klebsiella pneumoniae* capsular polysaccharide fragments to a nitrophenyl-ethylamine linker by reductive amination and attachment of the derivatized sugar using azo coupling.

U.S. Patent No. 4,057,685 by McIntire describes a *Escherichia coli* lipopolysccharide with reduced toxicity covalently coupled to a protein antigen by reaction with haloacyl halide.

U.S. Patent No. 4,356,170 by Jennings et al. describes the production of polysaccharide-protein conjugates by reductive amination.

U.S. Patent No. 4,673,574, 4,761,283 and 4,808,700 by Anderson describes the production of immunogenic conjugates comprising the reductive amination product of an immunogenic capsular polysaccharide fragment derived from the capsular polymer of *Streptococcus pneumoniae* or *H. influenzae* containing a reducing end prepared by means such as oxidative cleavage with periodate or by hydrolyses of a glycosidic linkage, with a bacterial toxin or toxoid as a protein carrier.

U.S. Patent No. 4,459,286 by Hillman et al. describes the preparation of a polysaccharide-protein conjugate by activation of the *H. influenzae* type b polysaccharide with cyanogen bromide, derivatization of the activated polysaccharide with the spacer molecule, 6-aminocaproic acid, and the conjugation of the major outer membrane protein of *Neisseria meningitidis* with a water soluble carbodiimide to form an amido type of linkage to the protein through a complex variety of linkages from the 6-amiriocaproic acid spacer to the polysaccharide.

U.S. Patent No. 4,965,338 by Gordon describes the production of a watersoluble covalent polysaccharide-diphtheria toxoid conjugate, wherein a pure *H*. *influenzae* type b polysaccharide is activated with cyanogen bromide and immediately mixed with diphtheria toxiod which has been derivatized with an ADH spacer.

U.S. Patent No. 4,663,160 by Tsay et al. describes a detoxified polysaccharide from a gram-negative bacteria covalently coupled to a detoxified protein from the same species of gram-negative bacteria by means of a 4-12 carbon moiety.

U.S. Patent No. 4,619,828 by Gordon et al describes conjugates between polysaccharide molecules from pathogenic bacteria such as *Haemophilus influenza* type *B, Streptococcus pneumoniae, Neisseria meningitidis* and *Escherichia coli* and T cell dependent antigens such as diphtheria and tetanus toxoids.

U.S. Patent No. 4,711,779 by Porro et al describes glycoprotein conjugate vaccines having trivalent immunogenic activity comprising antigenic determinants from the capsular polysaccharides of a gram-positive bacteria, as well as either CRM₁₉₇, tetanus toxoid, or pertusis toxin.

U.S. Patent No. 5,306,492 by Porro describes an oligosaccharide-carrier protein conjugate produced by reacting an oligosaccharide having a terminal reducing group with diaminomethane in the presence of pyridine borane such that reductive amination occurs, reacting the aminated oligosaccharide product with a molecule having two functional groups, and then reacting the activated oligosaccharide product with a carrier protein.

U.S. Patent No. 5,192,540 by Kuo et al describes a polysaccharide-protein conjugate comprising the reductive amination product of an oxidized polyribosyl-ribitol-phosphate polysaccharide fragment derived from the capsular polysaccharide of *Haemophilus influenzae* type b and the outer membrane protein of *Haemophilus influenzae* type b.

European publication No. EP 0747063 A2 describes a modified capsular polysaccharide containing multiple sialic acid derivatives and a heterobifunctional linker molecule linked to a carrier molecule. The linkers are used to N-alkylate up to about 5 sialic residues per polysaccharide. The remaining amino groups are then acylated with proprionic or acetic anhydride.

More efficient, higher yielding and simpler means of obtaining purified immunogenic polysaccharide-protein conjugates for large-scale production of immunogenic polysaccharide-protein conjugate vaccines are desirable.

Published PCT patent application WO 96/40239 (D1) describes modified meningococcal polysaccharide conjugate vaccines in which modified polysaccharides are conjugated to a protein carrier.

US Patent No. 5,576,002 (D2) describes modified meningococcal polysaccharide conjugate vaccines in which modified polysaccharides are conjugated to a protein carrier.

US Patent No. 4,727,136 (D3) describes modified meningococcal polysaccharide conjugate vaccines in which modified polysaccharides are conjugated to a protein carrier.

Jennings et al (1989, J Immunol. 142(10): 3585-91) (D4) describe a unique intermolecular bactericidal epitope involving the homosialopolysaccharide capsule on the cell surface of group B *Neisseria meningitidis* and *Escherichia coli* K1.

Fusco et al (1997, J Infect Dis. 175(2): 364-72) report the preclinical evaluation of a novel group B meningococcal conjugate vaccine that elicits bactericidal activity in both mice and nonhuman primates.

Granoff et al (1998, J Immunol. 160(10): 5028-36) describe bactericidal monoclonal antibodies that define unique meningococcal B polysaccharide epitopes that do not cross-react with human polysialic acid.

### SUMMARY OF THE INVENTION

The invention is an immunogenic β-propionamido-linked polysaccharide- and β-propionamido-linked oligosaccharide-protein conjugate.

It is an object of this invention to provide a method for preparing immunogenic β-propionamido-linked polysaccharide-protein conjugates which provide advantages over currently employed methodologies. It is a further object of this invention to provide pharmaceutical compositions, vaccines and other immunological reagents derived from the immunogenic β-propionamido-linked polysaccharide-protein conjugates.

A method of preparing an immunogenic polysaccharide-protein conjugate is provided which comprises de-N-acetylation of a polysaccharide or an oligosaccharide by base or enzymatic hydrolysis followed by N-acryloylation of the N-deacetylated polysaccharide. The N-acryloylated polysaccharide is directly coupled to a carrier protein to form the immunogenic β-propionamido-linked polysaccharide-protein conjugate.

Capsular and cell surface polysaccharides can be extracted according to this invention from either bacterial, yeast, or mammalian cell supernatants or directly from bacterial, yeast or mammalian cells by hydrolysis of the base labile bond that connects the polysaccharide to other cellular components or by enzymatic hydrolysis. A percentage of the N-acetyl groups removed by hydrolysis from the polysaccharide are replaced by N-acryloyl groups, which in turn, are directly coupled to protein to form the conjugate of the present invention.

An aspect of the invention provides oligosaccharides and polysaccharides that are directly coupled at multiple sites to protein(s).

Another aspect of the invention is a method of immunizing a mammal against bacterial or yeast infections or cancer, which comprises administration to the mammal an effective amount of the vaccine of the invention for prevention against infection from a disease causing organism or cancer.

An aspect of the invention is a method of eliciting the production of antibodies in mammals using the β-propionamido-linked polysaccharide-protein conjugates that protect the mammals against infection or disease.

Another aspect of the invention is immunoglobulin and isolated antibody elicited in response to immunization using β-propionamido-linked polysaccharide-protein conjugates. Such immunoglobulin and isolated antibody are useful as a therapeutics and as diagnostic reagents.

### BRIEF DESCRIPTION OF THE DRAWING

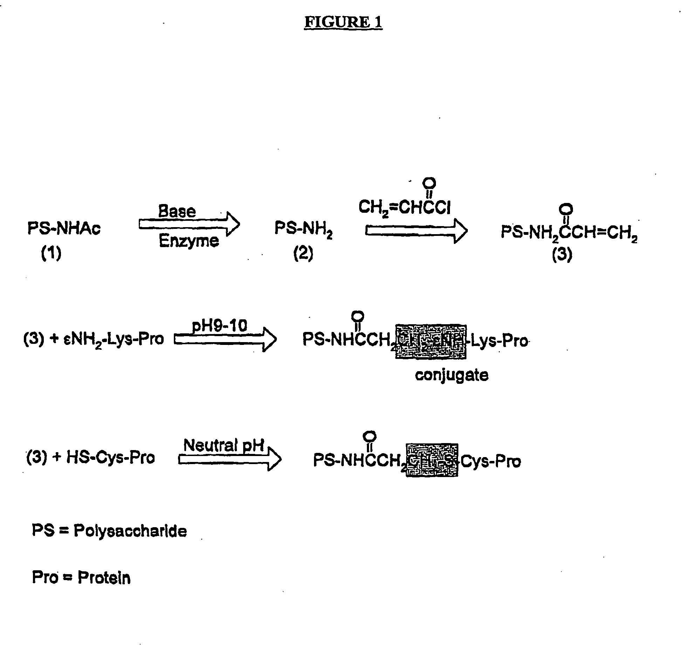

Figure 1. Schematic of the method of making the immunogenic β-propionamido-linked polysaccharide-proteinconjugates.

### DETAILED DESCRIPTION OF THE INVENTION

The invention is a novel polysaccharide-protein conjugate and oligosaccharide-protein conjugates useful as immunogens and vaccines against bacterial infections, yeast infections and as cancer therapeutics. Polysaccharides or oligosaccharides useful in forming immunogenic β-propionamido-linked polysaccharide-protein conjugates are derived from a source of polysaccharide or oligosaccharide which includes but is not limited to Gram (+) or Gram (-) bacteria, yeast, cancer cells or cancerous tissues and the like in which the polysaccharide or oligosaccharide serves as a virulence factor for the cell in evading host defense mechanisms. The polysaccharide-protein conjugates of the present invention are formed by direct coupling of the N-acryloylated polysaccharide with a protein by a Michael-type addition of nucleophilic sites on proteins.

Polysaccharides or oligosaccharides may be obtained from a variety of sources including gram-negative, gram-positive bacteria, yeast, cancer cells or recombinant forms of each using base or enzymatic hydrolysis of the bond that attaches the polysaccharide or oligosaccharide to the cellular components. Polysaccharide or oligosaccharide may be extracted from the organism or cell by contacting the organism or cell or a solution containing fragments of the organism or cell with an base or enzyme. Polysaccharide or oligosaccharide may then be recovered after basic or enzymatic hydrolysis by a variety of methods. Non-limiting examples of gram-positive bacteria and recombinant strains thereof for use according to this invention are Streptococci, Staphylococci, Enterococci, Bacillus, Corynebacterium, Listeria, Erysipelothrix, and Clostridium. Specifically, the use of Streptococci is more preferred and the use of group B Streptococci types Ia, Ib, II, III, IV, V, and VIII is most preferred. Non-limiting examples of gram-negative bacteria and recombinant strains thereof for use with this invention include *Haemophilus influenzae, Neisseria meningitides, Escherichia coli, Salmonella typhi, Klebsiella pneumoniae,* and *Pseudomonas aeruginosa*. Specifically, the use of *H. influenzae* type b, *N. meningitidis* types B, C, Y and W135, E. *coli* K1, and *E.coli* K92 are more preferred. Examples of yeast for use in the present invention include but are not limited to *Cryptococcus neoformans*. Examples of cancer cells or cancerous tissue for use in the present invention include but are not limited to small cell lung carcinoma, neuroblastomas, breast cancer, colon carcinoma, and the like.

A wide variety of conditions can be used for hydrolysis of the polysaccharide or oligosaccharide in either aqueous or organic solvent according to the invention by methods known in the art. The extent to which N-acetyl bonds of the carbohydrates are hydrolyzed can be controlled by the reaction conditions. In one embodiment, at least about 50% of the N-acetyl groups are removed by hydrolysis, preferably about 50% to about 100% are removed, more preferably about 90% or more of the native N-acetyl groups are removed. In a particular embodiment, about 95% or more of the N-acetyl groups are hydrolyzed from the polysaccharide by treatment with a hydrolysis reagent.

Capsular polysaccharides amenable to base extraction are those polysaccharides that lack any base-labile substituent that cannot be replaced, such as O-acetyl groups critical to immunogenicity. Other capsular polysaccharides amenable to base extraction are those lacking a phosphodiester bond and those lacking 4-linked uranic acid residues.

In a preferred embodiment for base hydrolysis, the CPS are extracted from group B Streptococci (GBS). In a most preferred embodiment the CPS are extracted from GBS types Ia, Ib, II, III, V and VIII.

In another preferred embodiment for base hydrolysis, the CPS are extracted from S. *pneumoniae.* In a more preferred embodiment for base hydrolysis the CPS are extracted from S. *pneumoniae* types III, IV and XIV.

In another preferred embodiment for base hydrolysis, the CPS are extracted from Neisseria or Escherichia bacteria. In a more preferred embodiment for base extraction, the CPS are extracted from *Neisseria meningitidis* types B, C, Y or W135, *Escherichia coli* K1 or *Escherichia coli* K92.

Polysaccharides amenable to enzymatic de-acetylation are those polysaccharides that lack any enzyme-labile substituent critical to immunogenicity in which the substituent cannot be replaced or substituted by an immunogenic moiety, these polysaccharides include but are not limited to GBS and the like.

### A. Preparation of the N-acryloylated polysaccharides

### 1. Deacetylation of Polysaccharides

### a). Starting Materials

Polysaccharide or oligosaccharide may be obtained using base hydrolysis or enzymatic hydrolysis from concentrated bacterial, yeast, mammalian cells or recombinant forms of these cells or from supernatants from homogenized cells or from conditioned medium using standard methods known in the art. The polysaccharide or oligosaccharide may be isolated and purified by standard methods known in the art. Isolated and purified polysaccharide or oligosaccharide from commercial sources may also be used as starting material.

Methods for isolation of the polysaccharide depend on the particular polysaccharide being used. A common method is the use of ionic detergent to complex with a charged polysaccharide. The complex is precipitated and isolated. The complex is then dissolved in a soluton of high ionic strength such as calcium chloride and the polysaccharide is then precipitated with ethanol

The isolated and purified polysaccharides and oligosaccharides obtained for use in this invention preferrably contain less than 1% nucleic acid and protein impurities for human use. Purities of 80-100% carbohydrate are often observed after purification due to the presence of inorganic salts.

### b). Base Hydrolysis

To remove the N-acetyl groups the purified polysaccharides or oligosaccharides can be treated with bases. Non-limiting examples of bases which may be used according to this invention are NaOH, KOH, LiOH, NaHCO₃, Na₂CO₃, K₂CO₃, KCN, Et₃N, NH₃, H₂N₂H₂, NaH, NaOMe, NaOEt or KOtBu. Bases such as NaOH, KOH, LiOH, NaH, NaOMe or KOtBu are most effectively used in a range of 0.5 N - 5.0 N. Bases such as NaHCO₃, Na₂CO₃, K₂CO₃ and KCN can be used in concentrations as high as their solubilities permit. Organic bases such as Et₃N can be used at medium to high (50-100%) concentrations as long as there is an agent such as water or alcohol to effect the hydrolysis. Bases such as NH₃ or H₂N₂H₂ can be used at nearly any concentration including 100%. Solvents such as water, alcohols (preferably C₁-C₄)_{,} dimethylsulfoxide, dimethylformamide or mixtures of these and other organic solvents can be used. Base solutions comprising water are most preferred.

The most effective pH range for removal of N-acetyl groups from the polysaccharide or oligosaccharide is from about 9 to about 14 with the optimal pH being around 12. The N-deacetylated polysaccharide thereafter is purified from residual reagents by ultrapurification using membranes or dialysis by standard methods known in the art.

### c). Enzymatic Hydrolysis

The enzyme, N-deacetylase may be used to enzymatically removed N-acetyl groups from a polysaccharide or oligosacctaride. In one embodiment, an N-deactylase enzyme useful in removal ofN-acetyl resides from polysaccharides or oligosaccharides is described in Refs 47, 48 and 49. In enzymatic hydrolysis, the polysaccharide or oligosaccharide and deacetylase enzyme are mixed with an appropriate enzyme buffer system under appropriate pH and temperature conditions and allowed to react for a period sufficient for removal ofN-acetyl groups. In one embodiment, polysaccharide and deacetylase enzyme are mixed with an appropriate enzyme buffer system, for example, 50 mM MES, 10 mM MnCl₂, pH 6.3 at 37°C for 60 minutes for formation of N-deacetylated polysaccharide. The reaction is stopped using an appropriate stopping solution for example 1 M monochloroacetic acid, 0.5 M NaOH, 2 M NaCl, or by dilution using an appropriate buffer solution.

### 2. N-Acryloylation of the Polysaccharide

The alkaline or enzymatic hydrolysis of the polysaccharide or oligosaccharide results in the removal ofN-acetyl groups from sialic acid and amino sugar residues of the polysaccharides or oligosaccharides. After hydrolysis, the polysaccharide or oligosaccharide is N-acryloylated to the extent desired by using a variety of acryloylating agents.

In one embodiment, the method comprises adding an acryloylating reagent to N-acrylolate an N-deacetylated polysaccharide or oligosaccharide. Examples of acryloylation reagents include but are not limited to acryloyl chloride, acryloyl anhydride, acrylic acid and a dehydrating agent such as DCC, CH₂CHCOCN the like, used in excess at a concentration of about 1 M. In a method of N-acryloylation of an N-deacetylated polysaccharide, the pH is adjusted and maintained at about 9 to about 11, preferably about pH 10 during the reaction. The temperature during reaction is about 2°C to about 8°C, preferably about 4°C. The reaction is carried out over a period of about 1 hour. The resulting N-acryloylated polysaccharide or N-acryloylated oligosaccharide is at least about 95% acryloylated or greater.

### B. Preparation of β-propionamido-linked polysaccharide-protein conjugates

The polysaccharide or oligosaccharide of this invention may be used to elicit antibody responses to a variety of gram-negative and gram-positive bacteria, yeast and cancers in an individual when conjugated to another immunogenic molecule such as a polypeptide or protein. Conjugation of the polysaccharide or oligosaccharide to the polypeptide converts the immune response to the polysaccharide or oligosaccharide which is typically T-cell independent to one which is T-cell dependent. Accordingly, the size of the polypeptide is preferably one which is sufficient to cause the conversion of the response from T-cell independent to T-cell dependent. It may by useful to use smaller polypeptides for the purpose of providing a second immunogen. The size of the protein carrier is typically from about 50,000 to about 500,000 M.W.

Preferred carrier proteins include, but are not limited to, tetanus toxoid, diphtheria toxoid, cholera toxin subunit B, *Neisseria meningitidis* outer membrane proteins, pneumolysoid, C-β protein from group B Streptococcus, non-IgA binding C-β protein from group B Streptococcus, *Pseudomonas aeruginosa* toxoid, pertussis toxoid, synthetic protein containing lysine or cysteine residues, and the like. The carrier protein may be a native protein, a chemically modified protein, a detoxified protein or a recombinant protein. Conjugate molecules prepared according to this invention, with respect to the protein component, may be monomers, dimers, trimers and more highly cross-linked molecules.

This invention provides the ability to produce conjugate molecules wherein the protein is linked to the polysaccharide or oligosaccharide through one or more sites on the polysaccharide or oligosaccharide. The size of the polysaccharide or oligosaccharide may vary greatly. One or a multiplicity of polysaccharides or oligosaccharides may cross-link with one or a multiplicity of protein. The conjugates of the present invention are preferably lattice structures. The points of attachment are between lysine or cysteine residues of the protein and the N-acryloyl groups of the polysaccharide or oligosaccharide.

In one method of forming a immunogenic polysaccharide-protein conjugate, an isolated polysaccharide (glycosaminoglycan) containing free amino groups or N-acyl groups (e.g. N-acetyl groups) in the sugar residues that constitute its repeating unit, is first treated hydrolyzed using base or enzyme to remove part or all of its N-acyl groups. The free amino groups are then N-acylated with an N-acryloylating reagent to form the N-acryloylated polysaccharide described above. The N-acryloylated polysaccharide is then directly coupled to protein under optimum conditions of pH, temperature and time to form an immunogenic β-propionamido-linked polysaccharide-protein conjugate.

In one embodiment, the method of conjugation is conducted at a pH above 9.0, preferably a pH of about 9.0 to about 10.0 for optimal reactivity of ε-free amino groups of lysine residues on the protein. In another embodiment, the method of conjugation is conducted at a neutral pH of about 7.0 for optimal reactivity of thiol (SH) groups of cysteine residues of the protein. The selection of pH for conducting the method of conjugation may be based on the number of reactive groups in a particular carrier protein. For example, a method using a protein composed of more reactive lysine residues as compared to cysteine residues is preferrably conducted at a basic pH. A method of conjugation using a protein composed of more reactive cysteine residues as compared to lysine residues is preferrably conducted at about a neutral pH.

The conjugation reaction may be conducted in buffered reagents including but not limited to a buffered reagent including carbonate/bicarbonate, borate buffer, phosphate and the like. The temperature of the conjugation reaction is at least about 25°C, preferably about 37 °C, for a period of preferably about 24 hours. The key reaction involves a 1,4-conjugate addition (Michael-type addition) of nucleophilic cysteine thiol groups or lysine ε-NH₂ groups on proteins with N-acryloylated sugar residues as described by Romanowska et al (46) which are present in the repeating-unit of the polysaccharide as shown in Figure 1. The resulting β-propionamido-linked polysaccharide-proteinconjugate has a polysaccharide to protein ratio of about 0.1 to about 0.6.

The glycosyl residues of the polysaccharide having N-acyl groups amenable to direct conjugation with cysteine and/or lysine residues on protein include, but are not limited to, glucosamine, galactosamine, mannosamine, fucosamine, sialic acids and the like. The polysaccharide may be derived from natural sources such as bacteria, yeast or cancer cells or from synthetic sources. Synthetic sources include chemical synthesis, enzymatic synthesis and chemoenzymatic synthesis. The synthesis may be *de novo* synthesis or the modification of natural carbohydrates. Naturally isolated carbohydrates can be modified by altering functional groups on carboyhydrate residues or by the addition or removal of carbohydrate residues.

The polysaccharide or oligosaccharide for use in preparing the β-propionamido-linked polysaccharide- and β-propionamido-linked oligosaccharide-protein conjugates of the present invention may vary in size for conjugation with a carrier protein. As defined herein, an oligosaccharide for use in the present invention comprises at least 10 sugar residues and preferably from 10 to about 50 sugar residues. A polysaccharide, as defined herein, is greater than 50 sugar residues and may be as large as about 600 or greater residues. In some cases, large constructs are desirable for enhancement of immunogenicity. The methods of this invention provide for the use of very large polysaccharides because many reactive sites can be introduced into a single polysaccharide. Another advantage of this method over the prior art is that the polysaccharide or oligosaccharide is not altered at a charged functional group which often interact with/or form part of the epitope crucial for immunity.

### C. Vaccines

This invention is also directed to vaccine preparations. According to this invention, the isolated β-propionamido-linked polysaccharide-protein conjugates described above may be used as an antigen to generate antibodies that are reactive against the polysaccharide or oligosaccharide and hence reactive against the organism or cell from which the polysaccharide or oligosaccharide was isolated. The vaccines of the present invention may be a combination or multi component vaccine further comprising in combination with the β-propionamido-linked polysaccharide-protein conjugate other components, including but not limited to Diphtheria-Tetanus-Pertusis (DTP), Tetanus-Diphtheria (Td), DTaP, a DTaP-Hib vaccine, a DTaP-IPV-Hib vaccine, and the like and combinations thereof, to provide a multifunctional vaccine useful in immunizing against a variety of diseases causing organisms or disease causing cells.

The vaccines of this invention may provide active or passive immunity. Vaccines for providing active immunity comprise an isolated and purified N-acryloylated polysaccharide or oligosaccharide conjugated to at least one antigenic peptide.

### D. Pharmaceutical compositions

The pharmaceutical compositions of this invention may comprise at least one polysaccharide-protein conjugate and pharmacologically acceptable carriers such as saline, dextrose, glycerol, ethanol or the like. In another embodiment the pharmaceutical composition comprises another immunogenic moiety, such as a peptide, or compositions comprising antibodies elicited by one of the CPS of this invention. The composition may also comprise adjuvants to enhance the immunological response of the recipient. Such adjuvants may be aluminum based such as alum or long chain alkyl adjuvants such as stearyl tyrosine (see U.S. Serial No. 583,372, filed 9/17/90; European Patent, EP 0 549 617 B1; Moloney et al. U.S. Patent No. 4,258,029), muramyl dipeptide (MDP) or derivative thereof, monophosphoryl lipid A (MPL), saponin (Quil-A) and the like. See also Jennings, et al. U.S Patent No. 5,683,699 and Paoletti, et al. J. Infectious Diseases 1997; 175:1237-9. The pharmaceutical composition may further comprise one or more additional immunogens including but not limited to Diphtheria-Tetanus-Pertusis (DTP), Tetanus-Diphtheria (Td), DTaP, DTaP-Hib, DTaP-IPV-Hib, and the like and combinations thereof. These pharmaceutical compositions are particularly useful as vaccines.

For eliciting passive immunity, the pharmaceutical composition may be comprised of polyclonal antibodies, or monoclonal antibodies, their derivatives or fragments thereof and recombinant forms thereof. The amount of antibody, fragment or derivative will be a therapeutically or prophylactically effective amount as determined by standard clinical techniques.

The pharmaceutical preparations of this invention may be introduced to an individual by methods known to be effective in the art. Intradermal, intraperitoneal, intravenous, subcutaneous, intramuscular, oral and intranasal are among, but not the only, routes of introduction.

The compositions of the invention may comprise standard carriers, buffers or preservatives known to those in the art which are suitable for vaccines including, but not limited to, any suitable pharmaceutically acceptable carrier, such as physiological saline or other injectable liquids. Additives customary in vaccines may also be present, for example stabilizers such as lactose or sorbitol and adjuvants to enhance the immunogenic response such as aluminum phosphate, hydroxide, or sulphate and stearyl tyrosine. The vaccines produced according to this invention may also be used as components of multivalent vaccines which elicit an immune response against a plurality of infectious agents.

Vaccines of the present invention are administered in amounts sufficient to elicit production of antibodies as part of an immunogenic response. The vaccine can be used parenternally to produce IgG and IgM antibodies or it can be delivered to the mucosal membranes to elicit IgA antibodies on the surface of tissues. Dosages may be adjusted based on the size, weight or age of the individual receiving the vaccine. The antibody response in an individual can be monitored by assaying for antibody titer or bactericidal activity and boosted if necessary to enhance the response. Typically, a single dose for an infant is about 10 µg of conjugate vaccine per dose or about 0.5 µg-20 µg/kilogram. Adults receive a dose of about 0.5 µg-20 µg/kilogram of the conjugate vaccine. For the CPS-protein conjugate vaccine, a typical dose is about 25 µg of each individual CPS per dose. That is, a vaccine against group B streptococcus may comprise 25 µg of each of the CPS form each of the nine serotypes.

### E. Antibodies

Antibodies directed against the polysaccharide may be generated by any of the techniques that are well known in the art. According to one approach, the antibodies may be generated by administering an isolated immunogenic β-propionamido-linked polysaccharide-protein conjugate into a host animal. The host animal may be, but is not limited to, rat, mouse, rabbit, non-human primate, or a human. Preferably, the host is human. In one embodiment, immunological responses may be increased by the use of adjuvants which are known in the art

Monoclonal antibodies directed against the polysaccharide may also be prepared by any of the techniques that are well known in the art. According to one method, cultures of hybridoma cell lines are used (Kohler and Milstein (1975) Nature 256:495-497). Monoclonal antibodies directed against the polysaccharide may be human monoclonal antibodies, chimeric monoclonal antibodies or humanized monoclonal antibodies made by any of the techniques that are well known in the art. According to one approach, chimeric monoclonal antibodies may be generated that have a non-human (e.g. mouse) antigen-binding domain combined with a human constant region. (Takeda et al. (1985) Nature 314:452). Humanized antibodies can be generated according to the procedures of Queen et al., U.S. Patent No. 5,585,089 and U.S. Patent No. 5,530,101. Single chain antibody may be constructed by methods known in the art (U.S. Patent No. 4,946,778; Davis, G.T. et al 1991 Biotechnology 9:165-169; Pluckthun, A. 1990 Nature 347:497-498). Constant region domains of the antibody may be modified by procedures known in the art (WO 89/07142)

Antibodies directed against the polysaccharide or oligosaccharide may be purified by any of the techniques that are well known in the art including, but not limited to immunoabsorption or immunoaffinity chromatography, or other chromatographic methods (e.g. HPLC). Antibodies may also be purified as immunoglobulin fractions from serum, plasma or cell culture medium.

Antibody molecules of this invention may be intact immunoglobulin molecules, substantially intact immunoglobulin molecules, or those portions of an immunoglobulin molecule, for example Fab fragments, that contain the antigen binding site. The antibody molecules may be of any class including IgG, IgM, and IgA.

Fragments of antibodies directed against the CPS may be generated by any of the techniques that are well known in the art. (Campbell (1985) Laboratory Techniques in Biochemistry and Molecular Biology, Vol. 13, Burdon, et al. (eds.), Elsevier Science Publishers, Amsterdam).

The antibody or antigen or antigen binding fragment thereof is useful as a therapeutic in providing passive protection against diseases caused by Gram (+), Gram (-) bacteria or yeasts. The antibody or antigen binding fragment thereof are also useful as a diagnostic reagent in standard immunoassays for the detection and/or identification of bacteria, yeast or cancer cells. The antibody may be supplied in kit form alone or with standard reagents for immunoassays.

In another embodiment of this invention, antibodies directed against the polysaccharide or oligosaccharide of this invention may be used as a pharmaceutical preparation in a therapeutic or prophylactic application in order to confer passive immunity from a host individual to another individual (i.e., to augment an individual's immune response against gram-negative or gram-positive bacteria or yeast or to provide a response in immuno-compromised or immuno-depleted individuals including AIDS patients). Passive transfer of antibodies is known in the art and may be accomplished by any of the known methods. According to one method, antibodies directed against the conjugates thereof of this invention are generated in an immunocompetent host ("donor") animal, harvested from the host animal, and transfused into a recipient individual. For example, a human donor may be used to generate antibodies reactive against the polysaccharide-protein conjugate of this invention. The antibodies may then be administered in therapeutically or prophylactically effective amounts to a human recipient in need of treatment, thereby conferring resistance in the recipient against bacteria which are bound by antibodies elicited by the polysaccharide component. (See Grossman, M. and Cohen, S. N., in "Basic and Clinical Immunology", 7th Ed., (Stites, D. P. and Terr, A. T. eds., Appleton & Lange 1991) Chapter 58 "Immunization".)

In certain cases the polysaccharide used with this invention may induce antibody which is cross-reactive with other pathogenic organisms and thus have ability in protecting against infection by these other bacteria.

### F. Diagnostic kits

In another embodiment, the CPS of this invention or derivatives or fragments thereof may be provided in diagnostic kits to indicate the presence of antibodies directed against bacteria, yeast or cancer cells. The presence of such antibodies can indicate prior exposure to the pathogen, and predict individuals who may be resistant to infection. The diagnostic kit may comprise at least one of the CPS of this invention or derivatives or fragments thereof, alone or conjugated to protein, and suitable reagents for the detection of an antibody reaction when the modified CPS or derivatives or fragments are mixed with a sample that contains antibody directed against gram-negative, gram-positive bacteria, yeast or cancer cells or cancer tissue. An antibody reaction may be identified by any of the methods described in the art, including but not limited to an ELISA assay. Such knowledge is important, and can avoid unnecessary vaccination.

Alternatively, the diagnostic kit may further comprise a solid support or magnetic bead or plastic matrix and at least one of the CPS of this invention or derivatives or fragments thereof.

In some cases, it may be preferred that the CPS or derivatives or fragments are labeled. Labeling agents are well-known in the art. For example, labeling agents include but are not limited to radioactivity, chemiluminescence, bioluminescence, luminescence, or other identifying "tags" for convenient analysis. Body fluids or tissues samples (e.g. blood, serum, saliva) may be collected and purified and applied to the diagnostic kit. The CPS, derivatives or fragments may be purified or non-purified and may be composed of a cocktail of molecules.

Solid matrices are known in the art and are available, and include, but are not limited to polystyrene, polyethylene, polypropylene, polycarbonate, or any solid plastic material in the shape of test tubes, beads, microparticles, dip-sticks, plates or the like. Additionally matrices include, but are not limited to membranes, 96-well micro titer plates, test tubes and Eppendorf tubes. In general such matrices comprise any surface wherein a ligand-binding agent can be attached or a surface which itself provides a ligand attachment site.

All publications, patents and articles referred to herein are expressly incorporated herein *in toto* by reference thereto. The following examples are presented to illustrate the present invention but are in no way to be construed as limitations on the scope of the invention. It will be recognized by those skilled in the art that numerous changes and substitutions may be made without departing from the spirit and purview of the invention.

### EXAMPLE 1

### Preparation of β-Propionamido-Linked Polysaccharide-Protein Carrier Conjugates

The following non-limiting examples describe the preparation of a series of clinically relevant polysaccharide-protein conjugates for vaccines against *Streptococcus pneumoniae* (type 14), Group B Streptococcus (GBS) type III and type II, and *E.coli* K1. All of the above polysaccharides used in this example are glycosaminoglycans that contain N-acetyl groups in one or more of the glycosyl residues that are constituents of their structural repeating-units.

### A. Depolymerization of type 14 pneumococcal polysaccharide

To increase its solubility the polysaccharide was first partially depolymerized by sonication. 200 mg of *Pneumococcal polysaccharide* type 14 (Lot NO 2020510, American Type Culture Collection) was dissolved in 20 ml of PBS and sonicated for 4 hours at 0°C with a Branson Sonifier Model 450. The resulting polysaccharide was dialyzed and lyophilized and then sized through a superdex 200 column equilibrated with phosphate buffered saline (PBS). Peak fractions were pooled and then dialyzed against d.i. water with Spectra/Por® Membrane MWCO:3,500. A yield of 157.5mg solid was obtained after lyophilization. The sonicated polysaccharide had an average molecular weight of about 50,000 as measured by SEC-MALLS with the miniDAWN (Wyatt Technology Corp., Santa Barbara, CA).

### B. De-N-Acetylation of type 14 pneumococcal polysaccharide

100 mg of sized type 14 pneumococcal polysaccharide was dissolved in 10 ml of 2N NaOH and then 10 mg of NaBH₄ was added to the reaction mixture. This mixture was heated at 100 °C for one hour and then cooled to room temperature. The N-deacetylated component was dialyzed against d.i. water with a Spectra/Por ® Membrane Membrane MWCO:3,500 and lyophilized to give 84 mg of white solid. The N-deacetylated polysaccharide was analysed by H¹-NMR at 500 MHz and was found to contain less than 5 percent residual N-acetyl groups.

### C. N-Acryloylation of the N-deacetylated type 14 Pneumococcal polysaccharide

84 mg of N-deacetylated type 14 Pneumococcal polysaccharide was dissolved in 4.2 ml of d.i. water. The solution, in an ice bath, was adjusted to pH 10 with 2 N NaOH. Then 420 µl of 1:1 v/v acryloyl chloride : dioxane was added and adjusted to pH 11 with 2 N NaOH. The reaction was allowed to stand for an additional hour at pH 11 to ensure the complete hydrolysis of esters which may have formed as a result of O-acylation. The solution was dialyzed and lyophilized to give 42 mg of dry powder. After analysis by 500 MHz H¹-NMR the polysaccharide was found to be over 95 percent N-acryloylated.

### D. Coupling of the type 14 N-acryloylated pneumococcal polysaccharide to tetanus toxoid monomer

22 mg of the type 14 N-acryloylated pneumococcal polysaccharide was dissolved in 1.1 ml of Carbonate/Bicarbonate pH 9.5 buffer. Tetanus toxoid monomer 22 mg was added to the reaction mixture. The reaction mixture was incubated overnight at 37 °C. The progress of the conjugation was analyzed with a Biologic system (Bio-Rad) equipped with a superose 12 column. Conjugation of polysaccharide to tetanus toxoid was indicated by the progressive increase in a peak, monitored by measurement of UV absorbance at 280 nm, eluting in the void volume of the column. After conjugation was complete, the solution was neutralized to pH 7. with 0.1N HCl and then dialyzed against PBS. The conjugate was purified by passage over a 1.6x60cm column of Superdex 200 PG (Pharmacia) and eluted with PBS containing 0.01 % thimerosal. Fractions corresponding to the void-volume peak were pooled. Carbohydrate and protein content in the conjugate were estimated by the phenol-sulfuric assay of Dubois et al. (51)and the Coomassie assay of Bradford (9).

Similar methods were used for GBS type II, type III as well as for the *E. coli* K1 and meningococcal C polysaccharides. The reaction conditions for each of these polysaccharides are tabulated below.

**TABLE 1**

| **E. De-N-Acetylation of GBS Type II and Type III Polysaccharide** | | | | | | | |
|---|---|---|---|---|---|---|---|
| | PS size^{*} (kD) | PS in mg | NaOH | NaBH₄ | Temp. | Reaction time | yield |
| GBSP II | 250 | 63 mg | 6 mL | 12 mg | 110°C | 6h | 63 mg |
| GBSP III | 110 | 50 mg | 5 mL | 10 mg | 110°C | 6h | 55 mg |

| | | | | | | | |
|---|---|---|---|---|---|---|---|
| *Determined by SEC-MALLS | | | | | | | |

**TABLE 2**

| **N-Acryloylation of GBS Type II and Type III polysaccharide** | | | | |
|---|---|---|---|---|
| | PS amount in mg | d.i. water | 1:1 v/v acryloyl chloride:dioxane | yield |
| GBSP II | 60 | 3 ml | 300 µl | 60 mg |
| GBSP III | 55 | 2.75 mL | 275 µl | 55 mg |

**TABLE 3**

| **Coupling of the GBS II and GBS III polysaccharide to Tetanus Toxoid Monomer** | | | | | |
|---|---|---|---|---|---|
| | PS in mg | TT in mg | Carbo/Bicarb buffer pH 9.5 | temperature | Incubation time |
| GBSP II | 10 | 10 | 0.5 mL | 37°C | overnight |
| GBSP III | 10.52 | 9.52 | 0.5 mL | 37°C | overnight |

### F. De-N-Acetylation of K1 polysaccharide

300 mg of K1 PS was dissolved in 15 mL of 2.0 N NaOH solution to which 150 mg of sodium borohydride was added. The solution was heated at 110°C for 6 hours, cooled down to room temperature and diluted with a 20-fold volume of dionized water. After diafiltation through an Amicon YM3 membrane with deionized water, the solution was lyophilized yielding 255 mg of N-deacetylated K1 PS. H¹-NMR at 500 MHz confirmed that complete N-deacetylation occurred.

### G. N-Acryloylation of K1 polysaccharide

To a 10 mL deionized water solution containing 250.mg of de-N-acetylated K1 PS, cooled in an ice bath, was added dropwise acryloyl chloride (Aldrich, Milwaukee,WI) solution, prepared by combining 1 mL of acryloyl chloride with a 1 mL of dioxane. The pH of the solution was maintained between 7.0 and 10.5 by the addition of 2 N sodium hydroxide solution. After completion of the addition, the pH was raised to 13 and maintained between 12.9 to 13.1 for I hour at room temperature. The pH of the solution was adjusted to 9.5 by the dropwise addition of 1 N HCL. The solution was diafiltrated with an Amicon YM3 membrane in a stircell with deionized water. The retentate was lyophilized to dryness, and the material (N-Acryloyl K1 PS) was stored at in a desiccator in a -20 C freezer. H-NMR at 500 MHz indicated that complete N-acryloylation took place during the reaction.

### H. β-Propionamido-Linked K1-rPorB conjugate (K1-rPorB I)

A solution containing 8.4 mg of N-Acryloyl K1 PS and 4.0 mg of recombinant *Neisseria meningitidis* PorB in 0.3 mL of 0.2 M borate, 0.05% Zwittergen™ 3,14 (Boehringer Mannhein) pH 9.5 was incubated at 37° C for 3 days. The conjugate was purified by size exclusion chromatography through a Superdex 200 preparative grade column, and eluted with PBS containing 0.01 % thimerosal. The fractions of uv-280 nm active signal eluting at or close to the void volume of the column were pooled and stored in the refrigerator. The conjugate was analysed for sialic acid and protein content by the resorcinol and Coomassie protein assays respectively.

### I. Preparation of Thiolated rPorB

To one ml of rPorB porin solution at a conc of 10 mg/ml in 0.25 M HEPES buffer of pH 8.5 containing 0.25 M sodium chloride and 0.05% zwittergent 3-14 was added 0.2 ml of 0.05 M N-succinimidyl 3-[2-pyridyldithio]propionate solution. The solution was mixed well and allowed to sit at RT for one hour. To the solution was added 0.06 ml of 1 M dithiothreitol solution in the same buffer. The solution was again mixed well and allowed to sit at RT for an additional two hours. The solution was diluted with 1.3 ml of 0.25 M HEPES buffer of pH 7.0 containing 0.25 M sodium chloride and 0.05% zwittergent 3-14 and loaded onto a Pharmacia PD-10 desalting column which had been pre-equilibrated with the same buffer. The column was eluted with the same buffer, and eluate was collected and concentrated with an Amicon Centricon 30 concentrator at 5,000 RPM for one hour. The retentate was collected and the protein concentration determined.

### H. Preparation of N-Acryloylated K1-S-rPorB Conjugate (K1-S-PorB)

To 0.17 ml of thiolated rPorB solution at a concentration of 25 mg/ml from above was added 9 mg of N-acryloylated K1 polysaccharide. The solution was mixed well and incubated in an oven of 37° C for 18 hours. The solution was purified through a Superdex 200 column (Pharmacia) with PBS as eluent. UV-280-nm-active fractions eluted at or close to the void volume of the column were combined. Analyses showed that the conjugate contained 25 ug/ml of polysaccharide and 188 ug/ml of protein.

### I. Preparation of N-Acryloylated GCMP-S-rPorB Conjugate (GCMP-S-rPorB)

Likewise, N-acryloylated GCMP-S-rPorB was prepared in a procedure comparable to the one described above for N-acryloylated K1-S-rPorB conjugate and found to contain 43 ug/ml of polysaccharide and 200 ug/ml of protein.

**TABLE 4**

| **Analytical Data for the Conjugates Described Above** | | | |
|---|---|---|---|
| | Protein Conc. µg/mL | PS Conc. µg/mL | Percent PS in conjugate |
| Pn14-TT (3) | 547 | 293 (1) | 35 |
| GBSII-TT(3) | 377 | 160 (2) | 30 |
| GBSIII-TT (3) | 365 | 115 (2) | 24 |
| K1-rPorB I (3) | 147 | 17 (2) | 10 |
| K1-rPorB II (4) | 406 | 41 (2) | 9 |
| K1-S-rPorB (3) | 188 | 25 (2) | 12 |
| GCMP-S-rPorB (3) | 200 | 43 (2) | 18 |

| | | | |
|---|---|---|---|
| (1) Total carbohydrate Dubois assay (2) resorcinol sialic assay (3) Prepared by direct coupling of the N-Acryloylated polysaccharide and the corresponding carrier protein (4) Control conjugate prepared by reductive amination of a periodate-oxidized N-Acryloylated K1 PS with rPorB | | | |

### EXAMPLE 2

### Immunogenicity and Potency of the β-Propionamido-Linked Polysaccharide-Protein Carrier Conjugates

### Preclinical evaluation of the conjugates in mice

Immunoassays: Serum antibody to each polysaccharide conjugate was measured by ELISA. The human serum albumin (HSA) (Sigma, St Louis, MO) conjugates used for ELISA assays were prepared by reductive amination. The oxidized polysaccharides were added to HSA followed by reductive amination with NaCNBH3. The conjugates were isolated by gel filtration chromatography, and stored freezed-dried at -70 °C. PS-specific antibody titers were determined by an ELISA as follows. Polystyrene, 96-well, flat-bottom microtiter plates (NUNC Polysorb) (Nunc, Naperville, IL) were coated with PS-HSA conjugates in PBS (0.01 M sodium phosphate, 0.15 M NaCL, pH 7.5 ) at 0.25 µg/well (100µL/ well) by incubating for 1 hour at 37°C, followed by a PBS-Tween (0.05% v/v Tween 20 in PBS) wash (5 times). All subsequent incubations were conducted at room temperature. PBS-Tween was used for all required washes. The coated plates were then blocked with PBS-BSA (0.5% w/v bovine serum albumin in PBS) for IgG ELISAs or 0.1% w/v Carnation nonfat dry milk for IgM ELISAs at 0.15 mL / well for 1 hour, followed by a wash. Sera were diluted 2-fold, in duplicate, in the plate at 100 µL/ well and incubated for 1 hour, followed by a wash. Antibody conjugate (peroxidase-labelled goat anti-mouse (Kirkegaard & Perry Lab, Gaithersburg, MD) was added at 100 µL/ well and incubated for 30 minutes, followed by a wash. A 1:1 dye and substrate solution (Kirkegaard & Perry TMB) and peroxide was added at 0.05mL/ well and incubated for 10 minutes. The peroxidase reaction was then stopped with 1 M H₃PO₄ at 0.05 mL/well, and the plate was read on a Molecular Devices Emax microplate reader (Molecular Devices, Menlo Park, CA) at a wavelength of 450 nm, using 650 nm as a reference wavelength. Background absorbances were determined in several no-serum control wells and averaged for each plate. For each serum dilution, the average background absorbance was substracted, and then duplicate serum absorbance values were averaged. A modified Scatchard plot was used for the subsequent data analysis, where the absorbance (y-axis) was plotted against the absorbance times the reciprocal dilution (x-axis) (ref). Under conditions allowing equilibrium and antibody excess, a straight line was obtained for each serum dilution series; this line was extrapolated to the x-axis for the determination of an antibody titer. A positive control serum, with a previously determined antibody titer, was used on each plate in order to provide a reference to which all sera were standardized, minimizing plate to plate and day to day variations. The results of these assays are shown in Tables 5, 6 and 7.

Opsonophagocytic assays (OP) : The opsonic activity of mice antisera to the Streptococcal B (GBS) and Pneumococcal conjugates was tested in an *in vitro* opsonophagocytic killing assay using the human promyelocytic leukemia HL-60 cell line (ATCC No. CCL 240). Briefly, 200 cfu of GBS type III strain M781 cells or pneumococcal type 14 strain were mixed in equal volume with serum antibodies and incubated under shaking 15 minutes at 35 °C in a 5% CO₂ incubator. Baby rabbit complement and HL-60 cells (5 x 10⁵) cultured 5 days in the presence of 90 mM DMF were added to the mixture and incubated at 37°C for 1 hour under shaking. Aliquots were removed for quantitative culture. Titers were determined by extrapolating the antibody dilution corresponding to fifty percent live bacteria. The results of these assays are shown in Table 5 for the pneumococcal type 14 conjugates and in Table 6 for the GBS type III conjugates.

*Serum* bactericidal assay (SBA) : Antibody-dependent complement-mediated bactericidal activity was measured in terms of the bactericidal titer, or reciprocal dilution, that provided 50% killing of the targeted bacteria. The complement in all sera was first incubated at 56 °C for 30 min. Then a 2-fold dilution series was established for each serum with GBSS in sterile 96-well U-bottom microtiter plates (Sigma), giving a final volume of 50 µL/ well. Infant rabbit serum complement (Pel-Freez, Brown Deer, WI) was diluted 1:1 with the working concentration of GBM bacteria (serotype 15 strain, 44/76) or Group C meningococcal C11 reference strain and 50 µL was added to each well containing the diluted serum, giving a final reaction mixture volume of 100 µL/ well. This reaction mixture, which contained 50% serum (heat-inactivated and diluted), 25% rabbit serum complement, and 25% bacteria (at working concentration), was incubated in a humidified incubator at 37 °C with 5% CO₂ for 60 min on a microtitration plate shaker (LKB-Wallac; pharmacia Biotech) at the fast speed.

All wells were then plated on chocolate Agar by spreading 30 µL/plate. Time zero bacterial samples were also plated. All plates were incubated overnight, as before. The colony-forming units (cfu) were then counted with an automated colony counter from Imaging Products International (Chantilly, VA), taking an average of three readings per plate. The reciprocal dilution, or titer, that gave 50% killing was read directly from a graph constructed where the x-axis represented the log10 value of the corresponding reciprocal dilution and the y-axis represented the percentage survival. The results of this assay are shown in Table 7.

**TABLE 5:**

| **Immunogenicity of Pneumococcal 14-Tetanus Toxoid Conjugates** | | | | | |
|---|---|---|---|---|---|
| Vaccine/ adjuvant | Elisa day0 | Elisa day28 | Elisa day38 | Elisa day59 | OP day59 |
| Control / Saline | <50 | 2,250 | 29,000 | 32,600 | 3,100 |
| Control / Alum | <50 | 18,200 | 99,000 | 265,000 | 25,000 |
| Pn14-TT/ Saline | <50 | 4,600 | 89,000 | 59,200 | 3,900 |
| Pn14-TT/ Alum | <50 | 27,000 | 185,000 | 251,000 | 26,000 |
| PBS/ Alum | <50 | <50 | <50 | <50 | <50 |

Control vaccine was a type 14 polysaccharide-tetanus toxoid conjugate prepared by reductive amination. Pn14-TT conjugate was the product of direct coupling between an N-Acryloylated type 14 pneumococcal polysaccharide and tetanus toxoid.

For this study groups of 10 CD1 mice (Charles River Laboratory) aged 6-8 weeks, were injected subcutaneously with 2.0 µg of conjugated polysaccharide on days 0, 28 and 38. The animals were bled on days 0, 28, 38 and exsanguinated on day 59. ELISAs were performed using Pn14 polysaccharide-HSA conjugate prepared by reductive amination. The ELISA titers reported in Table 5 represent total IgGs. The reported OP titers are against pneumococcal type 14 strain.

**TABLE 6:**

| **Immunogenicity of GBS Type III Conjugates** | | | | | |
|---|---|---|---|---|---|
| Vaccine/ Adjuvant | Elisa day 0 | Elisa day 21 | Elisa day 42 | Elisa day 52 | OP day 52 |
| Control conjugate/ Alum | <50 | 900 | 1,800 | 3,000 | 170 |
| GBS III-TT / Alum | <50 | 500 | 8,500 | 25,000 | 3,100 |
| PBS / Alum | <20 | <20 | <20 | <20 | <20 |

Control conjugate vaccine was a GBS type III-Tetanus toxoid conjugate prepared by reductive amination of a periodate oxidized GBS type III polysaccharide and tetanus toxoid. GBS III-TT conjugate was the product of direct coupling between an N-Acryloylated type III polysaccharide and tetanus toxoid.

For this study groups of 10 CD1 mice (Charles River Laboratory) aged 6-8 weeks, were injected subcutaneously with 2 µg of conjugated polysaccharide on days 0, 21, and 42. Mice were bled on days 0, 21, 42, and exsanguinated on day 52. ELISA titers were measured using a GBS type III polysaccharide coupled to human serum albumin, titers given in Table 6 represent total IgGs to the type III polysaccharide.

**TABLE 7:**

| **Immunogenicity of E. Coli K1 Conjugates** | | | | | |
|---|---|---|---|---|---|
| Vaccine / Adjuvant | Elisa day 0 | Elisa day 28 | Elisa day 42 | Elisa day 52 | SBA day 52 |
| K1-rPorB II /Alum Control Lot1 | <50 | 1,000 | 39,000 | 106,000 | 450 |
| K1-rPorB II / Alum Control Lot 2 | <50 | 450 | 124,000 | 250,000 | 1,000 |
| K1-rPorB I/ Alum Lot 1 | <50 | 220 | 27,000 | 96,000 | 810 |
| K1-rPorB I/ Alum Lot 2 | ND | ND | 24,000 | 71,000 | 3,800 |
| K1-S-rPorB/ Alum Lot 1 | ND | ND | 45,000 | 114,000 | 2,600 |
| K1-S-rPorB /Alum Lot2 | ND | ND | 41,000 | 94,000 | 1,700 |
| PBS / Alum | <50 | <50 | <50 | <50 | <50 |

Control vaccine (Kl-rPorB II) was the product of reductive amination between a periodate-oxidized N-Acryloylated K1 polysaccharide and tetanus toxoid. K1-rPorB I vaccine was the product of direct coupling of an N-Acryloylated K1 polysaccharide and tetanus toxoid. K1-S-rPorB was the product of direct coupling of the thiolated porin rPorB and the N-Acryloylated K1 polysaccharide.

For these studies groups of 10 CD1 mice (4-6 weeks old) from Charles River laboratory were immunized intraperitoneally on days 0, 28, and 42. Mice were bled on days 0, 28, 42, and then exsanguinated on day 52. ELISAs titers were measured using an N-Propionylated K1 polysaccharide coupled to human serum albumin. Titers shown in Table 7 represent total IgGs to the modified N-Propionylated K1 polysaccharide. Serum bactericidal activities (SBA) against *N.meningitidis* group B serotype 15 H44/76 strain, for the day 52 bleed, are also shown in Table 7.

**TABLE 8:**

| **Immunogenicity of GCMP Conjugates** | | |
|---|---|---|
| **Vaccine**/ **Adjuvant** | **ELISA day 38** | **SBA day 38** |
| GCMP-S-rPorB/ Alum Lot1 | 44,000 | 2,100 |
| GCMP-S-rPorB/ Alum Lot2 | 43,000 | 2,800 |
| PBS/ Alum | <50 | <50 |

GCMP-S-rPorB was the product of direct coupling between the N-Acryloylated group C meningococcal polysaccharide (GCMP) and the thiolated rPorB. For these animal studies groups of 10 outbred Swiss Webster female mice (6-8 weeks old) from HSD were injected s.c. with 2 µg of conjugated polysaccharide per dose on days 0 and 28. Animals were exsanguinated on day 38. ELISA titers to the group C polysaccharide are measured using a GCMP coupled to human serum albumin. Serum bactericidal titers are obtained using the meningococcal C11 reference strain.

### References

1. Anderson, P., G. Peter, R.B. Johnson, L.H. Wetterlow and D.H. Smith. 1972. Immunization of humans with polyribosylphosphate, the capsular antigen of Haemophilus influenzae type b. J.Clin.Invest. 51:39-44.
2. Avery, O.T. and W.F. Goebel. 1931. Chemo-immunological studies on conjugated carbohydrate-proteins V. The immunological specificity of an antigen prepared by combining the capsular polysaccharide of type 3 pneumococcus with foreign protein. J.Exp.Med. 54:437-447.
3. Baker, C.J. and D.L. Kasper. 1985. Group B streptococcal vaccines. Rev.Inf.Dis. 7:458-467.
4. Baker, C.J., M.A. Rench, M.S. Edwards, R.J. Carpenter, B.M. Hays and D.L. Kasper. 1988. Immunization of pregnant women with a polysaccharide vaccine of group B Streptococcus. N.Engl.J.Med. 319:1180-1185.
5. Baker, C.J., M.A. Rench and D.L. Kasper. 1990. Response to Type III polysaccharide in women whose infants have had invasive Group B streptococcal infection. New Engl.J.Med. 322:1857-1860.
6. Baltimore, R.S., D.L. Kasper and J. Vecchitto. 1979. Mouse protection test for group B Streptococcus type III. J.Infect.Dis. 140:81-86.
7. Bednar, B. and J.P. Hennessey. 1993. Molecular size analysis of capsular polysaccharide preparations from Streptococcus pneumoniae. Carbohyd.Res. 243:115-130.
8. Beri, R.G., J. Walker, E.T. Reese and J.E. Rollings. 1993. Characterization of chitosans via coupled size-exclusion chromatography and multiple-angle laser light-scattering technique. Carbohyd.Res. 238:11-26.
9. Bradford, M.M.. 1976. A Rapid and sensitive method for the quantitation of microgram quantities of protein utilizing the principle of protein-dye binding. Analyt.Biochem. 72:248-254.
10. D'Ambra, A.J., J.E. Baugher, P.E. Concannon, R.A. Pon and F. Michon. 1997. Direct and indirect methods for molar-mass analysis of fragments of the capsular polysaccharide of Haemophilus influenzae type b. Anal.Biochem. 250(2):228-236.
11. Dick, W.E., Jr. and M. Beurret. 1989. Glycoconjugates of bacterial carbohydrate antigens. p. 48-114. In: Conjugate Vaccines Contributions to Microbiology and Immunology. J.M. Cruse and R.E. Lewis,Jr., (eds) S.Karger, Basel.
12. Dillon, H.C., Jr., S. Khare and B.M. Gray. 1987. Group B streptococcal carriage and disease: A 6-year prospective study. J.Pediat. 110:31-36.
13. Goebel, W.F. and O.T. Avery. 1931. Chemo-immunological studies on conjugated carbohydrate-proteins IV. The synthesis of the p-aminobenzyl ether of the soluble specific substance of type 3 pneumococcus and its coupling with protein. J.Exp.Med. 54:431-436.
14. Gold, R., M.L. Lepow, I. Goldschneider, T.L. Draper and E.C. Gotschlich. 1975. Clinical evaluation of group A and group C meningococcal polysaccharide vaccines in infants. J.Clin.Invest. 56:1536-1547.
15. Gold, R., M.L. Lepow, I. Goldschneider and E.C. Gotschlich. 1977. Immune response of human infants to polysaccharide vaccines of Groups A and C Neisseria meningitides. J.Infect.Dis. 136S:S31-S35.
16. Gold, R.M., M.L. Lepow, I. Goldschneider, T.F. Draper and E.C. Gotschlich. 1978. Antibody responses of human infants to three doses of group A Neisseria meningitides vaccine administered at two, four and six months of age. J.Infect.Dis. 138:731-735.
17. Hennessey, J.P., B. Bednar and V. Manam. 1993. Molecular size analysis ofHaemophilus influenzae type b capsular polysaccharide. J.Liq.Chromat. 16:1715-1729.
18. Howard, J.G., G.H. Christie, B.M. Courtenay, E. Leuchars and A.J.S. Davies. 1971. Studies on immunological paralysis. VI. Thymic-independence of tolerance and immunity to type III pneumococcal polysaccharide. Cell.Immunol. 2:614-626.
19. Jennings, H.J., E. Katzenellenbogen, C. Lugowski and D.L. Kasper. 1983. Structure of the native polysaccharide antigens of type Ia and type Ib Group B Streptococcus. Biochemistry 22:1258-1263.
20. Jennings, H.J., K.-.G. Rosell and D.L. Kasper. 1980. Structural determination and serology of the native polysaccharide antigen of type III group B Streptococcus. Can.J.Biochem. 58:112-120.
21. Jennings, H.J., K.-.G. Rosell and D.L. Kasper. 1980. Structure and serology of the native polysaccharide antigen of type Ia group B Streptococcus. Proc.Nat.Acad.Sci.USA. 77:2931-2935.
22. Jennings, H.J., K.-.G. Rosell, E. Katzenellenbogen and D.L. Kasper. 1983. Structural determination of the capsular polysaccharide antigen of type II Group B Streptococcus. J.Biol.Chem. 258:1793-1798.
23. Jennings, H.J. and R.K. Sood. 1994. Synthetic glycoconjugates as human vaccines. p. 325-371. In: Y.C. Lee and R.T. Lee, Neoglycoconjugates: Preparation and applications. Academic Press, New York.
24. Kasper, D.L., C.J. Baker, R.S. Baltimore, J.H. Crabb, G. Schiffman and H.J. Jennings. 1979. Immunodeterminant specificity of human immunity to type III group B Streptococcus. J.Exp.Med. 149:327-339.
25. Knobloch, J.E. and P.N. Shaklee. 1997. Absolute molecular weight of low-molecular-weight heparins by size-exclusion chromatography with multiangle laser light scattering detection. Anal.Biochem. 245:231-241.
26. Lancefield, R.C.. 1933. A serological differentiation of human and other groups of haemolytic streptococci. J.Exp.Med. 57:571-595.
27. Lancefield, R.C.. 1938. A micro-precipitin technique for classifying hemolytic streptococci and improved methods for producing antigen. Proc.Soc.Exp.Biol.and Med. 38:473-478.
28. Lancefield, R.C., M. McCarty and W.N. Everly. 1975. Multiple mouse-protective antibodies directed against group B streptococci: Special reference to antibodies effective against protein antigens. J.Exp.Med. 142:165-179.
29. Madoff, L.C., L.C. Paoletti, J.Y. Tai and D.L. Kasper. 1994. Maternal immunization of mice with Group B streptococcal type III polysaccharide-beta C protein conjugate elicits protective antibody to multiple serotypes. J.Clin.Invest. 94:286-292.
30. Marques, M.B., D.L. Kasper, A. Shroff, F. Michon, H.J. Jennings and M.R. Wessels. 1994. Functional activity of antibodies to the group B polysaccharide of group B streptococci elicited by a polysaccharide-protein conjugate vaccine. Infect.Immun. 62:1593-1599.
31. Mäkelä, P.R.H., H. Peltola, H. Kayhty, et al. 1977. Polysaccharide vaccines of group A Neisseria meningitides and Haemophilus influenzae type b: A field trial in Finland. J.Infect.Dis. 136:543-50.
32. Michon, F., J.R. Brisson, A. Dell, D.L. Kasper and H.J. Jennings. 1988. Multiantennary group-specific polysaccharide of group B Streptococcus. Biochem. 27:5341-5351.
33. Peltola, A., H. Käyhty, A. Sivonen and P.R.H. Mäkelä. 1977. Haemophilus influenzae type b capsular polysaccharide vaccine in children: A double blind field study of 100,000 vaccines 3 months to 5 years of age in Finland. Pediatrics 60:730-737.
34. Peltola, H., P.R.H. Mäkelä, H. Jousimies, et al. 1977. Clinical efficacy of meningococcal group A vaccine in children three months to five years of age. N.Engl.J.Med. 297:686-691.
35. Reuter, G. and R. Schauer. 1994. Determination of sialic acids. p. 168-199. In: W.J. Lennarz and G.W. Hart, Methods in Enzymology Vol. 230 Techniques in Glycobiology. Academic Press, New York.
36. Robbins, J.B. and R. Schneerson. 1990. Polysaccharide-protein conjugates: Anew generation of vaccines. J.Infect.Dis. 161:821-832.
37. Smith, A.L. and J. Haas. 1991. Neonatal Bacterial Meningitis. p. 313-333. In: W.M. Scheld, R.J. Whitley and D.T. Durack, Infections of the Central Nervous System. Raven Press, Ltd., New York.
38. Tsunashima, T., K. Moro, B. Chu and T.-Y. Liu. 1978. Characterization of group C meningococcal polysaccharide by light-scattering spectroscopy. III. Determination of molecular weight, radius of gyration, and translational diffusional coefficient.. Biopolymers 17:251-265.
39. von Hunolstein, C., L. Nicolini, S. D'Ascenzi, C. Volpe, G. Alfarone and G. Orefici. 1993. Sialic acid and biomass production by Streptococcus agalactiae under different growth conditions. Appl.Microbiol.Biotechnol. 38:458-462.
40. Wessels, M.R., W.J. Benedi, H.J. Jennings, F. Michon, J.L. DiFabio and D.L. Kasper. 1989. Isolation and characterization of type IV group B Streptococcus capsular polysaccharide. Infect.Immun. 57:1089-1094.
41. Wessels, M.R., J.L. DiFabio, V.J. Benedi, et al. 1991. Structural determination and immunochemical characterization of the type V group B Streptococcus capsular polysaccharide. J.Biol.Chem. 266:6714-6719.
42. Wessels, M.R., L.C. Paoletti, D.L. Kasper, et al. 1990. Immunogenicity in animals of a polysaccharide-protein conjugate vaccine against type III group B Streptococcus. J.Clin.Invest. 86:1428-1433.
43. Wessels, M.R., L.C. Paoletti, A.K. Rodewald, et al. 1993. Stimulation of protective antibodies against type Ia and Ib group B streptococci by a type Ia polysaccharide-tetanus toxoid conjugate vaccine. Infect.Immun. 61:4760-4766.
44. Wessels, M.R., V. Pozsgay, D.L. Kasper and H.J. Jennings. 1987. Structure and immunochemistry of an oligosaccharide repeating unit of the capsule polysaccharide of Type III Group B Streptococcus: A revised structure for the Type III Group B streptococcal polysaccharide antigen. J.Biol.Chem. 262:8262-8267.
45. Wyle, S.A., M.S. Artenstein, B.L. Brandt, et al. 1972. Immunologic response of man to group B meningococcal polysaccharide vaccines. J.Infect.Dis. 126:514-522.
46. Romanowska, A., S.J. Meunier, F.D. Tropper, C.A. LaFerriere, and R. Roy. 1994. Michael Additions for Synthesis of Neoglycoproteins. Methods in Enzymology. Vol. 242:90-101.
47. Orellana, A. and C.B. Hirshberg. 1994. Molecular Cloning and Expression of a Glycosaminoglycan N-Acetylglucosaminyl N-Deacetylase/N-Sulfotransferase from a Heparin-producing Cell Line. J. Biol. Chem. Vol 269, No. 3 pp 2270-2276.
48. Cheung, W-F., I.Eriksson, M.Kusche-Gullberg, U. Lindahl, and L. Kjéllén, 1996. Expression of the Mouse Mastocytoma Glucosaminyl N-Deacetylase/N-sulfotransferase in Human Kidney 293 Cells Results in Increased N-Sulfation of Heparan Sulfate. Biochem 35: 5250-5256.
49. Kusche-Gullberg, M., I. Eriksson, D. Sandback Pikas, and L. Kjellen 1998. Identification and Expression in Mouse of Two Heparan Sulfate Glucosaminyl N-Deacetylase/N-Sulfotransferase Genes. J. Biol. Chem. Vol 273, No. 19: 11902-11907.
50. Finke, A., D. Bronner, A. V. Nikolaev, B. Jann, and K. Jann. 1991. Biosynthesis of the Escherichia coli K5 Polysaccharide, a Representative of Group II Capsular Polysaccharides: Polymerization In Vitro and Characterization of the Product. J. Bacteriology 173, No. 13: 4088-4094.
51. Dubois, M. et al 1965. Colormetric Method for the Determination of Sugars and Related Substance. Analytic. Chem. Vol. 28: 350-366.

## Claims

1. A polysaccharide-protein conjugate or oligosaccharide-protein conjugate comprising an N-propionated polysaccharide or N-propionated oligosaccharide directly coupled to a protein through β-position sites of propionate moieties of the N-propionated polysaccharide or N-propionated oligosaccharide; wherein the N-propionated polysaccharide or N-propionated oligosaccharide directly coupled to the protein elicits protective antibodies reactive against the N-propionated polysaccharide or N-propionated oligosaccharide; wherein the N-propionated polysaccharide or N-propionated oligosaccharide has at least 50% N-acryloyl groups; and wherein the protein is a bacterial protein or a synthetic protein containing lysine or cysteine residues.

2. A polysaccharide-protein conjugate or oligosaccharide-protein conjugate according to claim 1 wherein the polysaccharide or oligosaccharide is derived from a polysaccharide obtained from bacteria, yeast, cancer cells, or is chemically synthesized.

3. A polysaccharide-protein conjugate or oligosaccharide-protein conjugate according to claim 1 wherein the polysaccharide or oligosaccharide is derived from a polysaccharide obtained from *Escherichia coli,* Meningococcus, Pneumococcus, Streptococcus, Neisseria, Salmonella, Klebsiella, or Pseudomonas.

4. A polysaccharide-protein conjugate or oligosaccharide-protein conjugate according to claim 1 wherein the polysaccharide or oligosaccharide is derived from a polysaccharide obtained from group B *Streptococcus* selected from the group consisting of type Ia, type Ib, type II, type III, type V, type VIII, and combinations thereof.

5. A polysaccharide-protein conjugate or oligosaccharide-protein conjugate according to claim 3 wherein the polysaccharide or oligosaccharide is derived from a polysaccharide obtained from a *Meningococcus* group selected from the group consisting of group B, group C, group Y, group W135, and combinations thereof.

6. A polysaccharide-protein conjugate or oligosaccharide-protein conjugate according to claim 3 wherein the polysaccharide or oligosaccharide is derived from a polysaccharide obtained from *E*. *coli* K1, *E*. *coli* K92, Pneumococcus type 4, Pneumococcus type 14, Streptococcus group A, Streptococcus group C, or combinations thereof.

7. A polysaccharide-protein conjugate or oligosaccharide-protein conjugate according to claim 1 wherein the protein is selected from the group consisting of tetanus toxoid, diphtheria toxoid, a *Neisseria meningitidis* outer membrane protein, pneumolysoid, C-β protein from group B *Streptococcus* and non IgA-binding C-β protein from group B *Streptococcus.*

8. The polysaccharide-protein conjugate or oligosaccharide-protein conjugate according to claim 7 wherein the protein is recombinantly produced.

9. The polysaccharide-protein conjugate or oligosaccharide-protein conjugate according to claim 8 wherein the protein is recombinant *N. meningitidis* outer membrane protein.

10. A polysaccharide-protein conjugate or oligosaccharide-protein conjugate according to claim 1 wherein the polysaccharide or oligosaccharide comprises a glycosaminoglycan.

11. A polysaccharide-protein conjugate or oligosaccharide-protein conjugate according to claim 1 wherein the polysaccharide or oligosaccharide comprises glycosyl residues of a structural repeating unit having at least one free amino group or N-acyl group.

12. A polysaccharide-protein conjugate or oligosaccharide-protein conjugate according to claim 11 wherein the glycosyl residue is selected from the group consisting of glucosamine, galactosamine, mannosamine, fucosamine and sialic acid.

13. The polysaccharide-protein conjugate or oligosaccharide-protein conjugate according to claim 1 wherein the N-propionated polysaccharide or N-propionated oligosaccharide is directly coupled to an ε-free amino group of a lysine residue or a thiol group of a cysteine residue of the protein.

14. A conjugate according to claim 1 wherein the conjugate is selected from the group consisting of: a propionamido *Streptococcus pneumoniae* type 14 polysaccharide-tetanus toxoid conjugate, a propionamido group B streptococcus type III polysaccharide-tetanus toxoid conjugate, a propionamido group B Streptococcus type II polysaccharide-tetanus toxoid conjugate, a propionamido *E. coli* K1 polysaccharide-tetanus toxoid conjugate, and a propionamido meningococcal C polysaccharide-tetanus toxoid conjugate.

15. A polysaccharide-protein conjugate or oligosaccharide-protein conjugate comprising an N-propionated polysaccharide or N-propionated oligosaccharide directly coupled to a protein through β-position sites of propionate moieties of the N-propionated polysaccharide or N-propionated oligosaccharide; wherein the conjugate elicits protective antibodies reactive against the N-propionated polysaccharide or N-propionated oligosaccharide, wherein the conjugate is obtainable by a method comprising:
A) de-N-acetylating an isolated polysaccharide or oligosaccharide using a de-N-acetylating reagent to form a de-N-acetylated polysaccharide or a de-N-acetylated oligosaccharide,
B) N-acryloylating the de-N-acetylated polysaccharide or the de-N-acetylated oligosaccharide with an acryloylating reagent to form an N-propionated polysaccharide or an N-propionated oligosaccharide having at least 50% N-acryloyl groups, and
C) directly coupling through β-position sites of propionate moieties of the N-propionated polysaccharide or the N-propionated oligosaccharide to a bacterial protein or a synthetic protein containing lysine or cysteine residues to form the polysaccharide-protein conjugate or the oligosaccharide-protein conjugate.

16. The conjugate according to claim 15 wherein the polysaccharide or oligosaccharide is obtained from bacteria, yeast, or cancer cells or is chemically synthesized.

17. The conjugate of claim 15 wherein the coupling is conducted at a pH of about 7.0.

18. The conjugate of claim 15 wherein the coupling is conducted at a pH above 9.

19. The conjugate of claim 15 wherein the coupling is conducted in a reagent selected from the group consisting of phosphate buffer, bicarbonate buffer and borate buffer.

20. The conjugate of claim 15 wherein the de-N-acetylating reagent is a base or an enzyme and the acryloylating reagent is selected from the group consisting of N-acryloyl chloride, acryloyl anhydride, acrylic acid and a dehydrating agent.

21. A pharmaceutical composition comprising the conjugate according to claim 1 or 15 and a pharmaceutically acceptable carrier, wherein the pharmaceutical composition provides protective immunity against at least one member of a genus of an organism from which the polysaccharide or oligosaccharide component of the polysaccharide-protein conjugate or oligosaccharide-protein conjugate was obtained.

22. The pharmaceutical composition according to claim 21 further comprising an adjuvant.

23. The pharmaceutical composition according to claim 22 wherein the adjuvant is selected from the group consisting of alum and stearyl tyrosine.

24. The pharmaceutical composition according to claim 21 further comprising a second component, said second component selected from the group consisting of diphtheria-tetanus-pertussis (DTP), diphtheria-tetanus-acellular pertussis (DtaP), tetanus-diphtheria (Td), diphtheria-tetanus-acellular pertussis-*Haemophilus influenzae* type B (DTaP-Hib), diphtheria-tetanus-acellular pertussis-inactivated poliovirus-*Haemophilus influenzae* type B (DTaP-IPV-Hib), and combinations thereof.

25. An immunogen comprising the polysaccharide-protein conjugate or oligosaccharide-protein conjugate according to claim 1 or 15, wherein said immunogen elicits a polysaccharide-specific or an oligosaccharide-specific immune response when said immunogen is administered to a mammal for use as a vaccine.

26. The immunogen according to claim 25, wherein the immune response comprises generation of a polysaccharide-specific or an oligosaccharide-specific immunoglobulin.

27. The immunogen according to claim 26 wherein the immunoglobulin is IgG, IgM, IgA or combinations thereof.

28. A method of making a N-propionamido-linked polysaccharide-protein conjugate or a N-propionamido-linked oligosaccharide-protein conjugate, wherein the conjugate elicits protective antibodies reactive against the polysaccharide or oligosaccharide, the method comprising :
A) de-N-acetylating a polysaccharide or an oligosaccharide using a de-N-acetylating reagent to form a de-N-acetylated polysaccharide or de-N-acetylated oligosaccharide,
B) N-acryloylating the de-N-acetylated polysaccharide or de-N-acetylated oligosaccharide with an acryloylating reagent to form a N-propionated polysaccharide or a N-propionated oligosaccharide having at least 50% N-acryloyl groups, and
C) directly coupling through β-position sites of propionate moieties of the N-propionated polysaccharide or the N-propionated oligosaccharide to a bacterial protein or a synthetic protein containing lysine or cysteine residues to form the N-propionamido-linked polysaccharide-protein or N-propionamido-linked oligosaccharide-protein conjugate.

29. The method of claim 28, wherein the de-N-acetylating reagent is a base or enzyme.

30. The method of claim 28 wherein the de-N-acetylating reagent is selected from the group consisting of NaOH, KOH and LiOH.

31. The method of claim 28, wherein the acryloylating reagent is selected from the group consisting of acryloyl chloride, acryloyl anhydride, acrylic acid and a dehydrating agent.

32. The method of claim 28, wherein the polysaccharide or oligosaccharide is derived from a polysaccharide obtained from bacteria, yeast, cancer cells or is chemically synthesized.

33. The method of claim 28 wherein the polysaccharide or oligosaccharide is derived from a polysaccharide or oligosaccharide obtained from *Escherichia coli,* Meningococcus, Pneumococcus, Streptococcus, Neisseria, Salmonella, Klebsiella, or Pseudomonas.

34. The method of claim 28 wherein the protein is selected from the group consisting of tetanus toxoid, diphtheria toxoid, a neisserial outer membrane protein, pneumolysoid, and C-β protein from group B Streptococcus and non-IgA binding C-β protein from group B Streptococcus.

35. The method of Claim 34, wherein the protein is recombinantly produced.

36. A vaccine comprising the conjugate according to claim 1 or 15, wherein said vaccine provides protective immunity against at least one member of a genus of an organism from which the polysaccharide or oligosaccharide component of the polysaccharide-protein conjugate or oligosaccharide-protein conjugate was obtained.

37. A vaccine according to claim 36 wherein the organism is selected from the group consisting of bacteria and yeast.

38. A vaccine according to claim 37 wherein the bacteria is *Escherichia coli,* Meningococcus, Pneumococcus, Streptococcus, Neisseria, Salmonella, Klebsiella, or Pseudomonas.

39. A vaccine according to claim 36 further comprising a second immunogen in combination with the po!ysaccharide-protein conjugate or oligosaccharide-protein conjugate said second immunogen selected from the group consisting of DTP, DTaP, Td, DTaP, Hib, DTaP-IPV-Hib and combinations thereof.

40. Use of a polysaccharide-protein conjugate or oligosaccharide-protein conjugate according to Claim 1 or 15, wherein the polysaccharide or oligosaccharide in the conjugate is derived from a polysaccharide found in a disease causing organism or disease causing cell, in the manufacture of a vaccine for use in immunizing a mammal against said disease causing organism or disease causing cell.

41. Use according to claim 40 wherein the organism or cell is *Streptococcus pneumoniae.*

42. Use according to claim 40 wherein the organism or cell is a Group B Streptococcus.

43. Use according to claim 40 wherein the organism or cell is Group B *Neisseria meningitis*.

44. Use according to claim 40 wherein the organism or cell is Group C *Neisseria meningitis*.

45. Use according to claim 40 wherein the organism or cell is *Haemophilus influenzae* type B.

46. An isolated immunoglobulin or antigen binding fragment thereof elicited in response to the conjugate according to claim 1 or 15, said immunoglobulin or antigen binding fragment thereof specifically immunoreactive with an N-propionated polysaccharide or an N-propionated oligosaccharide and immunoreactive with a native N-acetylated polysaccharide from which the N-propionated polysaccharide or N-propionated oligosaccharide was derived.

47. The immunoglobulin or antigen binding fragment thereof according to claim 46, selected from the group consisting of IgG antibody, IgM antibody, IgA antibody and combinations thereof.

48. The immunoglobulin or antigen binding fragment thereof according to claim 47, wherein the immunoglobulin is an isolated IgG antibody.

49. The immunoglobulin or antigen binding fragment thereof according to claim 46 wherein the native N-acetylated polysaccharide is a component of bacteria, yeast or cancer cells.

50. The immunoglobulin or antigen binding fragment thereof according a claim 49 wherein the polysaccharide is derived from *Escherichia coli,* Meningococcus, Pneumococcus, Streptococcus, Neisseria, Salmonella, Klebsiella, or Pseudomonas.

51. The immunoglobulin or antigen binding fragment thereof according to claim 46 wherein the immunoglobulin is recombinantly produced.

52. Use of the immunoglobulin or antigen binding fragment thereof according to claim 46 to 51 in the manufacture of a medicament for passive immunization against said disease causing organism or disease causing cells.

53. The use according to claim 52 wherein the immunoglobulin is an isolated IgG antibody or antigen binding fragment thereof, or an isolated IgM antibody or antigen binding fragment thereof, or an isolated IgA antibody or antigen binding fragment thereof.

54. A polysaccharide-protein conjugate or oligosaccharide-protein conjugate according to any one of Claims 1 to 20 for use in medicine.

55. A polysaccharide-protein conjugate or oligosaccharide-protein conjugate according to Claim 1 or 15, wherein the polysaccharide or oligosaccharide in the conjugate is derived from a polysaccharide found in a disease causing organism or disease causing cell, for use in immunizing a mammal against said disease causing organism or disease causing cell.

56. A polysaccharide-protein conjugate or oligosaccharide-protein conjugate for use according to Claim 55, wherein the disease causing organism or cell is as defined in any of Claims 41 to 45.

## Patentansprüche

1. Polysaccharid-Protein-Konjugat oder Oligosaccharid-Protein-Konjugat, umfassend ein N-propioniertes Polysaccharid oder N-propioniertes Oligosaccharid, das direkt über β-Positionsstellen von Propionat-Einheiten des N-propionierten Polysaccharids oder N-propionierten Oligosaccharids an ein Protein gebunden ist, wobei das N-propionierte Polysaccharid oder N-propionierte Oligosaccharid, das direkt an das Protein gebunden ist, protektive Antikörper hervorruft, die gegen N-propionierte Polysaccharide oder N-propionierte Oligosaccharide reaktiv sind, wobei das N-propionierte Polysaccharid oder N-propionierte Oligosaccharid mindestens 50% N-Acryloylgruppen aufweist, und wobei das Protein ein bakterielles Protein oder ein synthetisches Protein, enthaltend Lysin- oder Cysteinreste, ist.

2. Polysaccharid-Protein-Konjugat oder Oligasaccharid-Protein-Konjugat nach Anspruch 1, wobei das Polysaccharid oder Oligosaccharid von einem Polysaccharid stammt, das von Bakterien, Hefe, Krebszellen erhalten wird, oder chemisch synthetisiert ist.

3. Polysaccharid-Protein-Konjugat oder Oligosaccharid-Protein-Konjugat nach Anspruch 1, wobei das Polysaccharid oder Oligosaccharid von einem Polysaccharid stammt, das von *Escherichia coli,* Meningococcus, Pneumococcus, Streptococcus, Neisseria, Salmonella, Klebsiella oder Pseudomonas erhalten wird.

4. Polysaccharid-Protein-Konjugat oder Oligosaccharid-Pratein-Konjugat nach Anspruch 1, wobei das Polysaccharid oder Oligosaccharid von einem Polysaccharid stammt, das von Gruppe B *Streptococcus,* ausgewählt aus der Gruppe, bestehend aus Typ Ia, Typ Ib, Typ II, Typ III, Typ V, Typ VIII und Kombinationen davon, erhalten wird.

5. Polysaccharid-Protein-Konjugat oder Oligosaccharid-Protein-Konjugat nach Anspruch 3, wobei das Polysaccharid oder Oligosaccharid von einem Polysaccharid stammt, das von einer *Meningococcus*-Gruppe, ausgewählt aus der Gruppe, bestehend aus Gruppe B, Gruppe C, Gruppe Y, Gruppe W135 und Kombinationen davon, erhalten wird.

6. Polysaccharid-Protein-Konjugat oder Oligosaccharid-Protein-Kvnjugat nach Anspruch 3, wobei das Polysaccharid oder Oligosaccharid von einem Polysaccharid stammt, das von *E. coli* K1, *E. coli* K92, Pneumococcus Typ 4, Pneumococcus Typ 14, Streptococcus Gruppe A, Streptococcus Gruppe C oder Kombinationen davon erhalten wird.

7. Polysaccharid-Protein-Konjugat oder Oligosaccharid-Protein-Konjugat nach Anspruch 1, wobei das Protein aus der Gruppe, bestehend aus Tetanustoxoid, Diphtherietoxoid, einem äußeren Membranprotein von *Neisseria meningitidis,* Pneumolysoid, C-β Protein von Gruppe B *Streptococcus* und nicht-IgA-bindendem C-β Protein von Gruppe B *Streptococcus* ausgewählt ist.

8. Polysaccharid-Protein-Konjugat oder Oligosaccharid-Protein-Konjugat nach Anspruch 7, wobei das Protein rekombinant hergestellt ist.

9. Polysaccharid-Protein-Konjugat oder Oligosaccharid-Protein-Konjugat nach Anspruch 8, wobei das Protein rekombinantes äußeres Membranprotein von *N*. *meningitidis* ist.

10. Polysaccharid-Protein-Konjugat oder Oligosaccharid-Protein-Konjugat nach Anspruch 1, wobei das Polysaccharid oder Oligosaccharid- ein Glycosaminogly-can umfaßt.

11. Polysaccharid-Protein-Konjugat oder Oligosaccharid-Protein-Konjugat nach Anspruch 1, wobei das Polysaccharid oder Oligosaccharid Glycosylreste einer sich strukturell wiederholenden Einheit umfaßt, die mindestens eine freie Aminogruppe oder N-Acylgruppe aufweist.

12. Polysaccharid-Protein-Konjugat oder Oligosaccharid-Protein-Konjugat nach Anspruch 11, wobei der Glycosylrest aus der Gruppe, bestehend aus Glucosamin, Galactosamin, Mannosamin, Fucosamin und Sialylsäure ausgewählt ist.

13. Polysaccharid-Protein-Konjugat oder Oligosaccharid-Protein-Konjugat nach Anspruch 1, wobei das N-propionierte Polysaccharid oder N-propionierte Oligosaccharid direkt an eine freie ε-Aminogruppe eines Lysinrestes oder eine Thiolgruppe eines Cysteinrestes des Proteins gebunden ist.

14. Konjugat nach Anspruch 1, wobei das Konjugat aus der Gruppe, bestehend aus: einem *Streptococcus pneumoniae* Typ 14 Propionamido-Polysaccharid-Tetanustoxoid-Konjugat, einem Gruppe B Streptococcus Typ III Propionamido-Polysaccharid-Tetanustoxoid-Konjugat, einem Gruppe B Streptococcus Typ II Propionamido-Polysaccharid-Tetanustoxoid-Konjugat, einem *E. coli* K1 Propionamid-Polysaccharid-Tetanustoxoid-Konjugat und einem Meningococcus C Propionamido-Polysaccharid-Tetanustoxoid-Konjugat, ausgewählt ist.

15. Polysaccharid-Protein-Konjugat oder Oligosaccharid-Protein-Konjugat, umfassend ein N-propioniertes Polysaccharid oder N-propioniertes Oligosaccharid, das direkt über β-Positionsstellen von Propionat-Einheiten des N-propionierten Polysaccharids oder N-propionierten Oligosaccharids an ein Protein gebunden ist, wobei das Konjugat protektive Antikörper hervorruft, die gegen das N-propionierte Polysaccharid oder N-propionierte Oligosaccharid reaktiv sind, wobei das Konjugat erhältlich ist durch ein Verfahren, umfassend:
A) De-N-acetylieren eines isolierten Polysaccharids oder Oligosaccharids unter Verwendung eines de-N-acetylierenden Reagenzes, um ein de-N-acetyliertes Polysaccharid oder ein de-N-acetyliertes Oligosaccharid zu erzeugen,
B) N-Acryloylieren des de-N-acetylierten Polysaccharids oder des de-N-acetylierten Oligosaccharids mit einem acryloylierenden Reagenz, um ein N-propioniertes Polysaccharid oder ein N-propioniertes Oligosaccharid zu erzeugen, das mindestens 50% N-Acryloylgruppen aufweist, und
C) direktes Binden an ein bakterielles Protein oder ein synthetisches Protein, enthaltend Lysin- oder Cysteinreste, über β-Positionsstellen von Propionat-Einheiten des N-propioniertes Polysaccharids oder des N-propionierten Oligosaccharids, um das Polysaccharid-Protein-Konjugat oder das Oligosaccharid-Protein-Konjugat zu erzeugen.

16. Konjugat nach Anspruch 15, wobei das Polysaccharid oder Oligosaccharid von Bakterien, Hefe oder Krebszellen erhalten wird, oder chemisch synthetisiert ist.

17. Konjugat nach Anspruch 15, wobei das Binden bei einem pH-Wert von etwa 7,0 durchgeführt wird.

18. Konjugat nach Anspruch 15, wobei das Binden bei einem pH-Wert oberhalb von 9 durchgeführt wird.

19. Konjugat nach Anspruch 15, wobei das Binden in einem Reagenz durchgeführt wird, ausgewählt aus der Gruppe, bestehend aus Phosphatpuffer, Bicarbonatpuffer und Boratpuffer.

20. Konjugat nach Anspruch 15, wobei das de-N-acetylierende Reagenz eine Base oder ein Enzym ist und das acryloylierende Reagenz aus der Gruppe, bestehend aus N-Acryloylchlorid, Acryloylanhydrid, Acrylsäure und einem Dehydratisierungsmittel, ausgewählt ist.

21. Pharmazeutische Zusammensetzung, umfassend das Konjugat nach Anspruch 1 oder 15 und einen pharmazeutisch verträglichen Träger, wobei die pharmazeutische Zusammensetzung protektive Immunität gegen mindestens ein Mitglied einer Gattung eines Organismus bereitstellt, von dem der Polysaccharid- oder Oligosaccharid-Bestandteil des Polysaccharid-Protein-Konjugats oder Oli-gosaccharid-Protein-Konjugats erhalten wurde.

22. Pharmazeutische Zusammensetzung nach Anspruch 21, weiter umfassend ein Adjuvans.

23. Pharmazeutische Zusammensetzung nach Anspruch 22, wobei das Adjuvans aus der Gruppe, bestehend aus Alum und Stearyltyrosin, ausgewählt ist.

24. Pharmazeutische Zusammensetzung nach Anspruch 21, weiter umfassend einen zweiten Bestandteil, wobei der zweite Bestandteil aus der Gruppe, bestehend aus Diphtherie-Tetanus-Pertussis (DTP), Diphtherie-Tetanus-azellulärem Pertussis (DtaP), Tetanus-Diphtherie (Td), Diphtherie-Tetanus-azellulärem Pertussis-*Haemophilus influenzae* Typ B (DTaP-Hib), Diphtherie-Tetanus-azellulärem Pertussis-inaktivierter Poliovirus-*Haemophilus influenzae* Typ B (DTaP-IPV-Hib) und Kombinationen davon, ausgewählt ist.

25. lmmunogen, umfassend das Polysaccharid-Protein-Konjugat oder Oligosaccharid-Protein-Konjugat nach Anspruch 1 oder 15, wobei das Immunogen eine Polysaccharid-spezifische oder eine Oligosacharid-spezifische Immunantwort hervorruft, wenn das Immunogen an ein Säugetier verabreicht wird, zur Verwendung als ein Impfstoff.

26. Immunogen nach Anspruch 25, wobei die Immunantwort das Erzeugen eines Polysaccharid-spezifischen oder eines Oligosaccharid-spezifischen Immunglobulins umfaßt.

27. Immunogen nach Anspruch 26, wobei das Immunglobulin IgG, IgM, IgA oder Kombinationen davon ist.

28. Verfahren zur Herstellung eines N-Propionamido-gebundenen Polysaccharid-Protein-Konjugats oder eines N-Propionamido-gebundenen Oligosaccharid-Protein-Konjugats, wobei das Konjugat protektive Antikörper, die gegen das Polysaccharid oder Oligosaccharid reaktiv sind, hervorruft, wobei das Verfahren umfaßt:
A) De-N-acetylieren eines Polysaccharids oder eines Oligosaccharids unter Verwendung eines de-N-acetylierenden Reagenzes, um ein de-N-acetyliertes Polysaccharid oder de-N-acetyliertes Oligosaccharid zu erzeugen,
B) N-Acryloylieren des de-N-acetylierten Polysaccharids oder de-N-acetylierten Oligosaccharids mit einem acryloylierenden Reagenz, um ein N-propioniertes Polysaccharid oder ein N-propioniertes Oligosaccharid zu erzeugen, das mindestens 50% N-Acryloylgruppen aufweist, und
C) direktes Binden an ein bakterielles Protein oder ein synthetisches Protein, enthaltend Lysin- oder Cysteinreste, über β-Positionsstellen von Propionat-Einheiten des N-propionierten Polysaccharids oder des N-propionierten Oligosaccharids, um das N-Propionamido-gebundene Polysaccharid-Protein oder N-Propionamido-gebundene Oligosaccharid-Protein-Konjugat zu erzeugen.

29. Verfahren nach Anspruch 28, wobei das de-N-acetylierende Reagenz eine Base oder Enzym ist.

30. Verfahren nach Anspruch 28, wobei das de-N-acetylierende Reagenz aus der Gruppe, bestehend aus NaOH, KOH und LiOH, ausgewählt ist.

31. Verfahren nach Anspruch 28, wobei das acryloylierende Reagenz aus der Gruppe, bestehend aus Acryloylchlorid, Acryloylanhydrid, Acrylsäure und einem Dehydratisierungsmittel, ausgewählt ist.

32. Verfahren nach Anspruch 28, wobei das Polysaccharid oder Oligosaccharid von einem Polysaccharid stammt, das von Bakterien, Hefe, Krebszellen erhalten wird oder chemisch synthetisiert wird.

33. Verfahren nach Anspruch 28, wobei das Polysaccharid oder Oligosaccharid von einem Polysaccharid oder Oligosaccharid stammt, das von *Escherichia coli*, Meningococcus, Pneumococcus, Streptococcus, Neisseria, Salmonella, Klebsiella oder Pseudomonas erhalten wird.

34. Verfahren nach Anspruch 28, wobei das Protein aus der Gruppe, bestehend aus Tetanustoxoid, Diphtherietoxoid, einem äußeren Membranprotein von Neisseria, Pneumolysoid und C-β Protein von Gruppe B Streptococcus und nicht-IgA-bindendem C-β Protein von Gruppe B Streptococcus, ausgewählt ist.

35. Verfahren nach Anspruch 34, wobei das Protein rekombinant hergestellt wird.

36. Impfstoff, umfassend das Konjugat nach Anspruch 1 oder 15, wobei der Impfstoff protektive Immunität gegen mindestens ein Mitglied einer Gattung eines Organismus, von dem der Polysaccharid- oder Oligosaccharidbestandteil des Polysaccharid-Protein-Konjugats oder Oligosaccharid-Protein-Konjugat erhalten wurde, bereitstellt.

37. Impfstoff nach Anspruch 36, wobei der Organismus aus der Gruppe, bestehend aus Bakterien und Hefe, ausgewählt ist.

38. Impfstoff nach Anspruch 37, wobei die Bakterien *Escherichia coli,* Meningococcus, Pneumococcus, Streptococcus, Neisseria, Salmonella, Klebsiella oder Pseudomonas sind.

39. Impfstoff nach Anspruch 36, weiter umfassend ein zweites Immunogen in Kombination mit dem Polysaccharid-Protein-Konjugat oder Oligosaccharid-Protein-Konjugat, wobei das zweite Immunogen aus der Gruppe, bestehend aus DTP, DTaP, Td, DTaP, Hib, DTaP-IPV-Hib und Kombinationen davon, ausgewählt ist.

40. Verwendung eines Polysaccharid-Protein-Konjugats oder Oligosaccharid-Protein-Konjugats nach Anspruch 1 oder 15, wobei das Polysaccharid oder Oligosaccharid in dem Konjugat von einem Polysaccharid stammt, das in einem Krankheiten verursachenden Organismus oder einer Krankheiten verursachenden Zelle gefunden wird, in der Herstellung eines Impfstoffs zur Verwendung in der Immunisierung eines Säugetiers gegen den Krankheiten verursachenden Organismus oder die Krankheiten verursachende Zelle.

41. Verwendung nach Anspruch 40, wobei der Organismus oder die Zelle *Streptococcus pneumoniae* ist.

42. Verwendung nach Anspruch 40, wobei der Organismus oder die Zelle ein Gruppe B Streptococcus ist.

43. Verwendung nach Anspruch 40, wobei der Organismus oder die Zelle Gruppe B *Neisseria meningitidis* ist.

44. Verwendung nach Anspruch 40, wobei der Organismus oder die Zelle Gruppe C *Neisseria meningitidis* ist.

45. Verwendung nach Anspruch 40, wobei der Organismus oder die Zelle *Haemophilus influenzae* Typ B ist.

46. Isoliertes Immunglobulin oder Antigen-bindendes Fragment davon, das als Antwort auf das Konjugat nach Anspruch 1 oder 15 hervorgerufen wurde, wobei das Immunglobulin oder Antigen-bindende Fragment davon spezifisch mit einem N-propionierten Polysaccharid oder einem N-propionierten Oligosaccharid immunreaktiv ist, und immunreaktiv mit einem nativen N-acetylierten Polysaccharid ist, von dem das N-propionierte Polysaccharid oder N-propionierte Oligosaccharid stammte.

47. Immunglobulin oder Antigen-bindendes Fragment davon nach Anspruch 46, ausgewählt aus der Gruppe, bestehend aus IgG-Antikörper, lgM-Antikörper, IgA-Antikörper und Kombinationen davon.

48. Immunglobulin oder Antigen-bindendes Fragment davon nach Anspruch 47, wobei das Immunglobulin ein isolierter IgG-Antikörper ist.

49. Immunglobulin oder Antigen-bindendes Fragment davon nach Anspruch 46, wobei das native N-acetylierte Polysaccharid ein Bestandteil von Bakterien, Hefe oder Krebszellen ist.

50. Immunglobulin oder Antigen-bindendes Fragment davon nach Anspruch 49, wobei das Polysaccharid von *Escherichia coli,* Meningococcus, Pneumococcus, Streptococcus, Neisseria, Salmonella, Klebsiella oder Pseudomonas stammt.

51. Immunglobulin oder Antigen-bindendes Fragment davon nach Anspruch 46, wobei das Immunglobulin rekombinant hergestellt wird.

52. Verwendung des lmmunglobulins oder Antigen-bindenden Fragments davon nach Anspruch 46 bis 51 in der Herstellung eines Medikaments zur passiven Immunisierung gegen den Krankheiten verursachenden Organismus oder die Krankheiten verursachende Zellen.

53. Verwendung nach Anspruch 52, wobei das Immunglobulin ein isolierter IgG-Antikörper oder Antigen-bindendes Fragment davon oder ein isolierter IgM-Antikörper oder ein Antigen-bindendes Fragment davon oder ein isolierter IgA-Antikörper oder Antigen-bindendes Fragment davon ist.

54. Polysaccharid-Protein-Konjugat oder Oligosaccharid-Protein-Konjugat nach einem der Ansprüche 1 bis 20 zur Verwendung in der Medizin.

55. Polysaccharid-Protein-Konjugat oder Oligosaccharid-Protein-Konjugat nach Anspruch 1 oder 15, wobei das Polysaccharid oder Oligosaccharid in dem Konjugat von einem Polysaccharid stammt, das in einem Krankheiten verursachenden Organismus oder einer Krankheiten verursachenden Zelle gefunden wird, zur Verwendung in der Immunisierung eines Säugetiers gegen den Krankheiten verursachenden Organismus oder die Krankheiten verursachende Zelle.

56. Polysaccharid-Protein-Konjugat oder Oligosaccharid-Protein-Konjugat, zur Verwendung nach Anspruch 55, wobei der Krankheiten verursachende Organismus oder die Zelle wie in einem der Ansprüche 41 bis 45 definiert ist.

## Revendications

1. Conjugué polysaccharide-protéine ou conjugué oligosaccharide-protéine comprenant un polysaccharide N-propioné ou un oligosaccharide N-propioné couplé directement à une protéine par l'intermédiaire de sites en position β de radicaux propionate du polysaccharide N-propioné ou de l'oligosaccharide N-propioné ; où le polysaccharide N-propioné ou l'oligosaccharide N-propioné couplé directement à la protéine déclenche des anticorps protecteurs réactifs contre le polysaccharide N-propioné ou l'oligosaccharide N-propioné ; où le polysaccharide N-propioné ou l'oligosaccharide N-propioné a au moins 50 % des groupes N-acryloylés ; et où la protéine est une protéine bactérienne ou une protéine synthétique contenant des résidus de lysine ou de cystéine.

2. Conjugué polysaccharide-protéine ou conjugué oligosaccharide-protéine selon la revendication 1, dans lequel le polysaccharide ou l'oligosaccharide est dérivé d'un polysaccharide obtenu à partir de bactéries, de levures, de cellules cancéreuses ou est synthétisé chimiquement.

3. Conjugué polysaccharide-protéine ou conjugué oligosaccharide-protéine selon la revendication 1, dans lequel le polysaccharide ou l'oligosaccharide est dérivé d'un polysaccharide obtenu à partir de *Escherichia coli,* d'un méningocoque, d'un pneumocoque, d'un streptocoque, d'une neisseria, d'une salmonelle, d'une klebsielle ou d'un pseudomonas.

4. Conjugué polysaccharide-protéine ou conjugué oligosaccharide-protéine selon la revendication 1, dans lequel le polysaccharide ou l'oligosaccharide est dérivé d'un polysaccharide obtenu à partir d'un streptocoque du groupe B choisi dans le groupe constitué par le type Ia, le type Ib, le type II, le type III, le type V, le type VIII et de combinaisons de ceux-ci.

5. Conjugué polysaccharide-protéine ou conjugué oligosaccharide-protéine selon la revendication 3, dans lequel le polysaccharide ou l'oligosaccharide est dérivé d'un polysaccharide obtenu à partir d'un groupe de méningocoques choisi dans le groupe constitué par le groupe B, le groupe C, le groupe Y, le groupe W135 et des combinaisons de ceux-ci.

6. Conjugué polysaccharide-protéine ou conjugué oligosaccharide-protéine selon la revendication 3, dans lequel le polysaccharide ou l'oligosaccharide est dérivé d'un polysaccharide obtenu à partir de *E. coli* K1, de *E. coli* K92, d'un pneumocoque de type 4, d'un pneumocoque de type 14, d'un streptocoque du groupe A, d'un streptocoque du groupe C ou de combinaisons de ceux-ci.

7. Conjugué polysaccharide-protéine ou conjugué oligosaccharide-protéine selon la revendication 1, dans lequel la protéine est choisie dans le groupe constitué par l'anatoxine tétanique, l'anatoxine diphtérique, une protéine de la membrane externe de *Neisseria meningitidis*, une pneumolysine, une protéine C-β provenant d'un streptocoque du groupe B et une protéine C-β ne liant pas les IgA provenant d'un streptocoque du groupe B.

8. Conjugué polysaccharide-protéine ou conjugué oligosaccharide-protéine selon la revendication 7, dans lequel la protéine est produite de manière recombinante.

9. Conjugué polysaccharide-protéine ou conjugué oligosaccharide-protéine selon la revendication 8, dans lequel la protéine est une protéine recombinante de la membrane externe de *N. meningitidis.*

10. Conjugué polysaccharide-protéine ou conjugué oligosaccharide-protéine selon la revendication 1, dans lequel le polysaccharide ou l'oligosaccharide comprend un glycosaminoglycane.

11. Conjugué polysaccharide-protéine ou conjugué oligosaccharide-protéine selon la revendication 1, dans lequel le polysaccharide ou l'oligosaccharide comprend des résidus de glycosyle d'une unité répétitive structurale ayant au moins un groupe amino ou N-acyle libre.

12. Conjugué polysaccharide-protéine ou conjugué oligosaccharide-protéine selon la revendication 11, dans lequel le résidu de glycosyle est choisi dans le groupe constitué de glucosamine, galactosamine, mannosamine, fucosamine et acide sialique.

13. Conjugué polysaccharide-protéine ou conjugué oligosaccharide-protéine selon la revendication 1, dans lequel le polysaccharide N-propioné ou l'oligosaccharide N-propioné est couplé directement à un groupe amino ε-libre d'un résidu de lysine ou à un groupe thiol d'un résidu de cystéine de la protéine.

14. Conjugué selon la revendication 1, où le conjugué est choisi dans le groupe constitué par : un conjugué propionamido polysaccharide de *Streptococcus pneumoniae* de type 14-anatoxine tétanique, un conjugué propionamido polysaccharide d'un streptocoque de type III du groupe B-anatoxine tétanique, un conjugué propionamido polysaccharide d'un streptocoque de type II du groupe B-anatoxine tétanique, un conjugué propionamido polysaccharide de *E*. *coli* K1-anatoxine tétanique et un propionamido polysaccharide de méningocoque C-anatoxine tétanique.

15. Conjugué polysaccharide-protéine ou conjugué oligosaccharide-protéine comprenant un polysaccharide N-propioné ou un oligosaccharide N-propioné couplé directement à une protéine par l'intermédiaire de sites en position β de radicaux propionate du polysaccharide N-propioné ou de l'oligosaccharide N-propioné ; où le conjugué déclenche des anticorps protecteurs réactifs contre le polysaccharide N-propioné ou l'oligosaccharide N-propioné, où le conjugué peut être obtenu par un procédé comprenant :
A) la dés-N-acétylation d'un polysaccharide ou d'un oligosaccharide isolé en utilisant un réactif de dés-N-acétylation pour former un polysaccharide dés-N-acétylé ou un oligosaccharide dés-N-acétylé,
B) la N-acryloylation du polysaccharide dés-N-acétylé ou de l'oligosaccharide dés-N-acétylé avec un réactif d'acryloylation pour former un polysaccharide N-propioné ou un oligosaccharide N-propioné ayant au moins 50 % des groupes N-acryloylés, et
C) le couplage direct par l'intermédiaire de sites en position β des radicaux propionate du polysaccharide N-propioné ou de l'oligosaccharide N-propioné à une protéine bactérienne ou une protéine synthétique contenant des résidus de lysine ou de cystéine pour former le conjugué polysaccharide-protéine ou le conjugué oligosaccharide-protéine.

16. Conjugué selon la revendication 15, dans lequel le polysaccharide ou l'oligosaccharide est obtenu à partir de bactéries, de levures ou de cellules cancéreuses ou est synthétisé chimiquement.

17. Conjugué selon la revendication 15, où le couplage est conduit à un pH d'environ 7,0.

18. Conjugué selon la revendication 15, où le couplage est conduit à un pH supérieur à 9.

19. Conjugué selon la revendication 15, où le couplage est conduit dans un réactif choisi dans le groupe constitué par un tampon phosphate, un tampon bicarbonate et un tampon borate.

20. Conjugué selon la revendication 15, où le réactif de dés-N-acétylation est une base ou une enzyme et le réactif d'acryloylation est choisi dans le groupe constitué de chlorure de N-acryloyle, d'anhydride d'acryloyle, d'acide acrylique et d'un agent déshydratant.

21. Composition pharmaceutique comprenant le conjugué selon la revendication 1 ou 15 et un support pharmaceutiquement acceptable, où la composition pharmaceutique fournit une immunité protectrice contre au moins un membre d'un genre d'un organisme à partir duquel le composant polysaccharidique ou oligosaccharidique du conjugué polysaccharide-protéine ou du conjugué oligosaccharide-protéine a été obtenu.

22. Composition pharmaceutique selon la revendication 21, comprenant en outre un adjuvant.

23. Composition pharmaceutique selon la revendication 22, dans laquelle l'adjuvant est choisi dans le groupe constitué d'alun et de stéaryl-tyrosine.

24. Composition pharmaceutique selon la revendication 21, comprenant en outre un second composant, ledit second composant choisi dans le groupe constitué de DTP (diphtérie-tétanos-coqueluche), DTaP (diphtérie-tétanos-coqueluche acellulaire), Td (tétanos-diphtérie), DTaP-Hib (diphtérie-tétanos-coqueluche acellulaire-*Haemophilus influenzae* type B), DTaP-IPV-Hib (diphtérie-tétanos-coqueluche acellulaire-poliovirus inactivé-*Haemophilus influenzae* type B) et des combinaisons de ceux-ci.

25. Immunogène comprenant le conjugué polysaccharide-protéine ou le conjugué oligosaccharide-protéine selon la revendication 1 ou 15, où ledit immunogène déclenche une réponse immunitaire spécifique du polysaccharide ou spécifique de l'oligosaccharide lorsque ledit immunogène est administré à un mammifère pour une utilisation en tant que vaccin.

26. Immunogène selon la revendication 25, où la réponse immunitaire comprend la génération d'une immunoglobuline spécifique du polysaccharide ou spécifique de l'oligosaccharide.

27. Immunogène selon la revendication 26, où l'immunoglobuline est une IgG, une IgM ou une IgA ou des combinaisons de celles-ci.

28. Procédé de fabrication d'un conjugué polysaccharide à liaison N-propionamido-protéine ou d'un conjugué oligosaccharide à liaison N-propionamido-protéine, où le conjugué déclenche des anticorps protecteurs réactifs contre le polysaccharide ou l'oligosaccharide, le procédé comprenant :
A) la dés-N-acétylation d'un polysaccharide ou d'un oligosaccharide en utilisant un réactif de dés-N-acétylation pour former un polysaccharide dés-N-acétylé ou un oligosaccharide dés-N-acétylé,
B) la N-acryloylation du polysaccharide dés-N-acétylé ou de l'oligosaccharide dés-N-acétylé avec un réactif d'acryloylation pour former un polysaccharide N-propioné ou un oligosaccharide N-propioné ayant au moins 50 % des groupes N-acryloylés, et
C) le couplage direct par l'intermédiaire de sites en position β des radicaux propionate du polysaccharide N-propioné ou de l'oligosaccharide N-propioné à une protéine bactérienne ou une protéine synthétique contenant des résidus de lysine ou de cystéine pour former le conjugué polysaccharide à liaison N-propionamido-protéine ou le conjugué oligosaccharide à liaison N-propionamido-protéine.

29. Procédé selon la revendication 28, dans lequel le réactif de dés-N-acétylation est une base ou une enzyme.

30. Procédé selon la revendication 28, dans lequel le réactif de dés-N-acétylation est choisi dans le groupe constitué de NaOH, KOH et LiOH.

31. Procédé selon la revendication 28, dans lequel le réactif d'acryloylation est choisi dans le groupe constitué de chlorure d'acryloyle, d'anhydride d'acryloyle, d'acide acrylique et d'un agent déshydratant.

32. Procédé selon la revendication 28, dans lequel le polysaccharide ou l'oligosaccharide est dérivé d'un polysaccharide obtenu à partir de bactéries, de levures ou de cellules cancéreuses ou est synthétisé chimiquement.

33. Procédé selon la revendication 28, dans lequel le polysaccharide ou l'oligosaccharide est dérivé de *Escherichia coli,* d'un méningocoque, d'un pneumocoque, d'un streptocoque, d'une neisseria, d'une salmonelle, d'une klebsielle ou d'un pseudomonas.

34. Procédé selon la revendication 28, dans lequel la protéine est choisie dans le groupe constitué par l'anatoxine tétanique, l'anatoxine diphtérique, une protéine de la membrane externe de neisseria, une pneumolysine et une protéine C-β provenant d'un streptocoque du groupe B et une protéine C-β ne liant pas les IgA provenant d'un streptocoque du groupe B.

35. Procédé selon la revendication 34, dans lequel la protéine est produite de manière recombinante.

36. Vaccin comprenant le conjugué selon la revendication 1 ou 15, où ledit vaccin fournit une immunité protectrice contre au moins un membre d'un genre d'un organisme à partir duquel le composant polysaccharidique ou oligosaccharidique du conjugué polysaccharide-protéine ou du conjugué oligosaccharide-protéine a été obtenu.

37. Vaccin selon la revendication 36, où l'organisme est choisi dans le groupe constitué de bactéries et de levures.

38. Vaccin selon la revendication 37, où la bactérie est *Escherichia coli,* un méningocoque, un pneumocoque, un streptocoque, une neisseria, une salmonelle, une klebsielle ou un pseudomonas.

39. Vaccin selon la revendication 36, comprenant en outre un second immunogène en combinaison avec le conjugué polysaccharide-protéine ou le conjugué oligosaccharide-protéine, ledit second immunogène choisi dans le groupe constitué de DTP, DTaP, Td, DTaP-Hib, DTaP-IPV-Hib et de combinaisons de ceux-ci.

40. Utilisation d'un conjugué polysaccharide-protéine ou d'un conjugué oligosaccharide-protéine selon la revendication 1 ou 15, où le polysaccharide ou l'oligosaccharide dans le conjugué est dérivé d'un polysaccharide trouvé dans un organisme provoquant une maladie ou une cellule provoquant une maladie, dans la fabrication d'un vaccin pour une utilisation dans l'immunisation d'un mammifère contre ledit organisme provoquant une maladie ou ladite cellule provoquant une maladie.

41. Utilisation selon la revendication 40, où l'organisme ou la cellule est *Streptococcus pneumoniae.*

42. Utilisation selon la revendication 40, où l'organisme ou la cellule est un streptocoque du groupe B.

43. Utilisation selon la revendication 40, où l'organisme ou la cellule est *Neisseria meningitidis* du groupe B.

44. Utilisation selon la revendication 40, où l'organisme ou la cellule est *Neisseria meningitidis* du groupe C.

45. Utilisation selon la revendication 40, où l'organisme ou la cellule est *Haemophilus influenzae* type B.

46. Immunoglobuline isolée ou fragment de liaison de l'antigène de celle-ci déclenchée en réponse au conjugué selon la revendication 1 ou 15, ladite immunoglobuline ou ledit fragment de liaison de l'antigène de celle-ci spécifiquement immunoréactive/immunoréactif avec un polysaccharide N-propioné ou un oligosaccharide N-propioné et immunoréactive/immunoréactif avec un polysaccharide N-acétylé natif à partir duquel le polysaccharide N-propioné ou l'oligosaccharide N-propioné a été dérivé.

47. Immunoglobuline ou fragment de liaison de l'antigène de celle-ci selon la revendication 46, choisie dans le groupe constitué des anticorps IgG, des anticorps IgM, des anticorps IgA et des combinaisons de ceux-ci.

48. Immunoglobuline ou fragment de liaison de l'antigène de celle-ci selon la revendication 47, où l'immunoglobuline est un anticorps IgG isolé.

49. Immunoglobuline ou fragment de liaison de l'antigène de celle-ci selon la revendication 46, où le polysaccharide N-acétylé natif est un composant de bactéries, de levures ou de cellules cancéreuses.

50. Immunoglobuline ou fragment de liaison de l'antigène de celle-ci selon la revendication 49, où le polysaccharide est dérivé de *Escherichia coli,* d'un méningocoque, d'un pneumocoque, d'un streptocoque, d'une neisseria, d'une salmonelle, d'une klebsielle ou d'un pseudomonas.

51. Immunoglobuline ou fragment de liaison de l'antigène de celle-ci selon la revendication 46, où l'immunoglobuline est produite de manière recombinante.

52. Utilisation de l'immunoglobuline ou du fragment de liaison de l'antigène de celle-ci selon les revendications 46 à 51, dans la fabrication d'un médicament destiné à une immunisation passive contre ledit organisme provoquant une maladie ou lesdites cellules provoquant une maladie.

53. Utilisation selon la revendication 52, où l'immunoglobuline est un anticorps IgG isolé ou un fragment de liaison de l'antigène de celui-ci, ou un anticorps IgM isolé ou un fragment de liaison de l'antigène de celui-ci, ou un anticorps IgA isolé ou un fragment de liaison de l'antigène de celui-ci.

54. Conjugué polysaccharide-protéine ou conjugué oligosaccharide-protéine selon l'une quelconque des revendications 1 à 20 pour une utilisation en médecine.

55. Conjugué polysaccharide-protéine ou conjugué oligosaccharide-protéine selon la revendication 1 ou 15, où le polysaccharide ou l'oligosaccharide dans le conjugué est dérivé d'un polysaccharide trouvé dans un organisme provoquant une maladie ou une cellule provoquant une maladie, pour une utilisation dans l'immunisation d'un mammifère contre ledit organisme provoquant une maladie ou ladite cellule provoquant une maladie.

56. Conjugué polysaccharide-protéine ou conjugué oligosaccharide-protéine destiné à une utilisation selon la revendication 55, où l'organisme provoquant une maladie ou la cellule provoquant une maladie est tel(le) que défini(e) dans l'une quelconque des revendications 41 à 45.
